# EUROPEAN PATENT SPECIFICATION

(11) **EP 2 693 944 B1**
(45) Date of publication and mention of the grant of the patent: **20.06.2018**
(21) Application number: 12768677.2
(22) Date of filing: 03.04.2012
(51) Int. Cl.: A61B 5/117, A61B 5/1455, G06K 9/00, A61B 5/00, A61B 5/02, A61B 5/024, G06F 21/32, A61B 5/0205, A61B 5/026, A61B 5/0402, A61B 5/091

(54) **METHOD FOR BIOMETRIC IDENTITY CONFIRMATION**
VERFAHREN ZUR BIOMETRISCHEN IDENTITÄTSBESTÄTIGUNG
PROCÉDURE POUR UNE CONFIRMATION D'IDENTITÉ BIOMÉTRIQUE

(30) Priority: 04.04.2011 US 201113079219; 27.06.2011 US 201113169603; 13.12.2011 US 201161570109 P; 20.01.2012 US 201261589084 P
(43) Date of publication of application: 12.02.2014
(73) Proprietor: Lifeloc Technologies, Inc., Wheat Ridge, Colorado 80033 (US)
(72) Inventor: PHILLIPS, Brian, K., Lakewood CO 80220 (US); WILSON, Geoffrey, A., Roseburg OR 97471 (US)
(74) Representative: Smallman, Clint Guy
(86) International application number: PCT/US2012/032014
(87) International publication number: WO 2012/138663

(56) References cited:
- US-A1- 2003 135 097
- US-A1- 2003 135 097
- US-A1- 2007 038 330
- US-A1- 2007 177 770
- US-A1- 2007 287 892
- US-A1- 2009 253 130
- US-A1- 2010 108 425
- US-A1- 2010 152 600
- US-A1- 2010 204 592
- US-A1- 2010 331 716
- US-A1- 2011 009 762

## Description

### BACKGROUND OF THE INVENTION

**Field of the Invention.** The present invention relates generally to the field of biometric identity confirmation. More specifically, the present invention discloses a system for biometric identity confirmation based on both spirometric data and pulse wave data for a test subject.

**Background of the Invention.** Biometric identification is the process of recognizing or rejecting an unknown person as a particular member of a previously characterized set, based on biological measurements. The ideal biometric characterization is specific to the individual, difficult to counterfeit, robust to metabolic fluctuations, insensitive to external conditions, easily measured, and quickly processed.

Fingerprint, retinal, iris, and facial scans are well-known biometric identification techniques relying on image processing. Images are two-dimensional, requiring sophisticated and computationally intensive algorithms, the analysis of which is often complicated by random orientation and variable scaling. Voice recognition is an example of biometric identification amenable to time series analysis, an inherently simpler one-dimensional process.

The simplest biometric identifiers can be expressed as a single parameter, such as height or weight. Single parameter identifiers have been the only quantitative means of identification throughout most of history. The price of simplicity is the loss of specificity, and in the case of weight, the lack of constancy over time. Nevertheless, single-parameter biometrics remain effective identifying factors, as is obvious from their continued use.

Identity tracking/confirmation is the process of following the whereabouts of a known subject moving unpredictably among similar individuals, perhaps with deceptive intent. Tracking/confirmation is somewhat simpler than identification, because it merely requires distinguishing the subject from all others rather than distinguishing every individual from every other, and because continuous rather than episodic data are available. Biometric identity tracking/confirmation is the continuous verification that a body-mounted sensor has remained on the subject, and has not been surreptitiously transferred to an impostor. For the purposes of this disclosure the term "biometric identification" should be broadly construed to encompass both biometric identification in its narrower sense, as described above, and identity tracking/confirmation.

The repeatability of spirometry data provides a basis for its use in identity tracking /confirmation. In general terms, spirometry is a pulmonary function testing technique for measuring airflow and lung capacity, also known as lung volume. Various spirometric parameters, along with the flow-volume loop described in the next section, are promising for client identity confirmation (CIC) because they vary widely among individuals, but are fairly stable from measurement to measurement for a specific individual over a typical service period, and resist counterfeiting. It is apt to compare spirometric parameters with the familiar biometric human height - they have similar specificities (ratio of population range to individual stability) and immunities to deception.

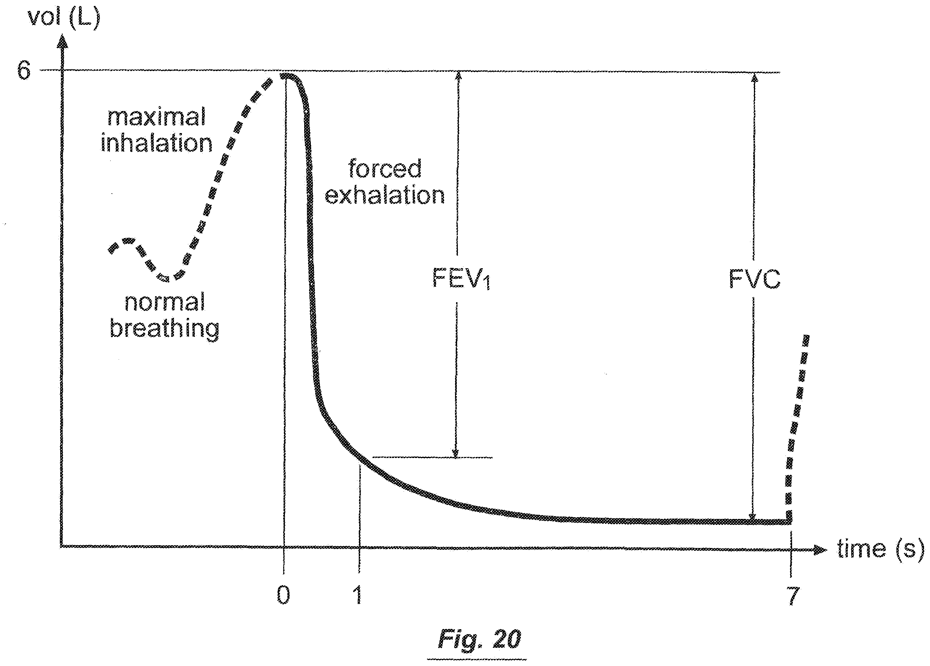

The spirogram is a plot of lung volume versus time during a maximal inhalation and exhalation, which can diagnose airway obstructions and constrictions, inadequate diaphragm function, or thoracic cage abnormalities. Figure 20 is a schematic spirogram of a forced vital capacity test, consisting of a maximal inhalation followed by a forced exhalation. Inhalation is depicted with a dotted line, because the invention measures only exhaled breath. This spirogram plots lung volume versus time over one cycle of maximal inhalation and forced exhalation. Spirometry is a mature clinical diagnostic, and was standardized decades ago by the American Thoracic Society (ATS).

Among the several measures of lung volume, the forced vital capacity (FVC), defined as the difference between the volumes of maximum inhalation and exhalation, and the forced expiratory volume in the first second (FEV₁) are particularly suit the invention. Because FVC measures the maximum air volume expellable in a single breath, it is physiologically impossible for the subject to overblow, so a measurement significantly greater than the baseline established during sensor "enrollment" indicates collusion with a cohort with more FVC than the subject. A measurement significantly lesser than the baseline indicates deception, involving either collusion with a cohort with less FVC than the subject, or the subject deliberately reserving exhalation to avoid a deep lung sample. FEV₁, which is rather independent of FVC, may be the most reproducible flow parameter. US 2003/135097 A1 provides methods and apparatuses useful for verification of identity, which may be used for a variety of purposes such as a prerequisite to entry of secured facilities, use of restricted information systems, and conduct of business or legal activities, as well as identification of unknown individuals and confirmation of the identity of known individuals. Biometric techniques are used that are based on dynamic physiological signals such as heart rate variability and/or respiration processes. The time derivative of the spirogram gives the airflow versus time. The most prominent feature of this curve is the peak expiratory flow (PEF), which is correlated to but distinct from FEV₁. The PEF's chief utility is that an operational shortfall relative to the enrollment baseline during operation indicates the subject is not maximally exhaling, possibly with deceptive intent.

The flow volume loop (FVL) is a plot of lung volume versus airflow, thus eliminating time as an explicit variable, while retaining implicit dynamical information. As the term "loop" implies, the FVL is cyclical or nearly so. The FVL encompasses all the spirometric parameters discussed above, therefore the shape of a client's FVL must be at least as specific as the spirometric parameter set. As the FVL may be the easiest representation of spirometric data to interpret and the most informative, it is incorporated into the example embodiment of the invention below.

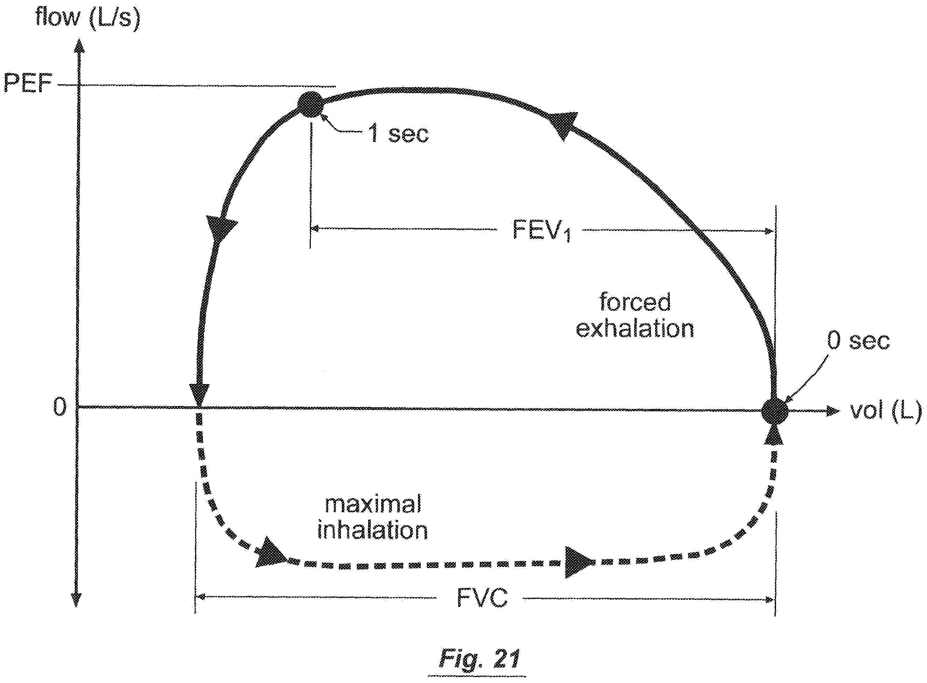

Figure 21 is a schematic FVL of a forced vital capacity test, with exhalation consisting of the positive-flow portion of the loop (solid line), proceeding counterclockwise from peak volume at time zero. The FVL plots airflow versus lung volume over one or more cycles of maximal inhalation and forced exhalation. Time has been eliminated as an explicit variable, but advances in the counterclockwise direction indicated by arrowheads. By convention, the time origin is placed at the lung capacity maximum. One can read the PEF and FVC directly from the FVL plot in Figure 21. The exhaled volume can be found by integrating flow over time, and FEV₁ = V(0)-V(1).

Diagnosis is the chief clinical application of the spirogram and related plots. Consequently, the primary aim in the medical literature is to establish norms for spirometric parameters and FVLs, according to sex, age, height, and so on. The secondary aim is sometimes to identify an ailment according to the nature of its deviation from the norm.

Furthermore, clinicians are also concerned with repeatability, to best discern borderline abnormalities and therapeutic progress. The ATS has defined repeatability as the largest and median results of three maneuvers (recorded exhalations) must differ by no more than 0.2 liters, for both FVC and FEV₁. Considering that a ballpark value for either parameter is two liters, the spirometry session is deemed unrepeatable if either ΔFVC is more than 10% of FVC, or ΔFEV₁ is more than 10% of FEV₁.

Repeatability appears readily achievable. In one study of 18,000 adult patients, only 5% of the patients were unable to match their highest FEV₁ within 150 ml, and half matched their two largest FEV₁'s within 58 ml, or 3% of FEV₁ ("Repeatability of Spirometry in 18,000 Adult Patients", P. L. Enright et al., Am. J. Respir. Crit. Care Med. 169, pp. 235-238 (2004)). This result was irrespective of patient sex or age. Other groups have performed repeatably - a study of 852 children reported 87.9% achievement of ΔFVC less than 5% ("Forced expiratory manoeuvres in children: do they meet ATS and ERS criteria for spirometry?", H. G. M. Arets et al., Eur. Respir. J. 18, pp. 655-660 (2001)). In a study of 7,101 sufferers of chronic pulmonary obstructive disease (COPD), approximately 86% met the criterion of less than 50 mL absolute and 10% relative, for either ΔFEV₁ or ΔFVC ("Variability of Spirometry in Chronic Obstructive Pulmonary Disease", L. B. Herpel et al., Am. J. Respir. Crit. Care Med. 173, pp. 1106-1113 (2006)). Other studies have reported good repeatability with children, the elderly and asthmatics

### SUMMARY OF THE INVENTION

This invention provides a testing method for biometric identity confirmation. The testing method where acquiring the spirometric data includes the subject exhaling into a testing unit and exerting labial pressure on a mouthpiece of the testing unit, and acquiring the pulse waveform data includes simultaneously performing lip pulse photoplethysmography. A processor analyzes spirometric data from the spirometric sensor and simultaneous lip pulse photoplethysmography data from the pulse sensor during the breath sample, together with stored subject characterization data for a known subject to confirm whether the identity of the test subject matches the known subject. A communications link enables the processor to communicate to an external station whether the identity of the test subject matches the known subject. For example, this can be used to control access to a secure facility or computer, authenticate the identity of a party in a financial transaction, or confirm the identity of the subject of an alcohol monitoring test.

During an initial enrollment mode, pulse wave and spirometric data for a known subject are used to generate subject characterization data for the known subject. During a subsequent identity authentication mode, pulse wave and spirometric data for a test subject are analyzed using the subject characterization data to confirm whether the identity of the test subject matches the known subject.

These and other advantages, features, and objects of the present invention will be more readily understood in view of the following detailed description and the drawings.

### BRIEF DESCRIPTION OF THE DRAWINGS

The present disclosure can be more readily understood in conjunction with the accompanying drawings, in which:
FIG. 1 is a simplified cross-sectional view of the portable testing unit 10.
FIG. 2 is a system block diagram of the portable testing unit 10.
FIG. 3 is a top perspective view of the portable testing unit 10.
FIG. 4 is a flowchart of the enrollment mode of the present invention.
FIG. 5 is a flowchart of the identity authentication mode
FIG. 6 is a flowchart of the "acquire trial" procedure for pulse wave data.
FIG. 7 is a flowchart of the "acquire trial" procedure for spirometric data.
FIG. 8 is a more detailed flowchart of the enrollment mode for the preferred embodiment.
FIG. 9 is a more detailed flowchart of an alternative embodiment of the enrollment mode.
FIG. 10 is a more detailed flowchart of the identity authentication mode of the preferred embodiment
FIG. 11 is a system diagram for the present invention.
FIG. 12(a) is a flowchart of the enrollment mode
FIG. 12(b) is a flowchart of the operational mode
FIG. 13 is a system diagram of an embodiment using blood pressure and pulsatile blood volume as the pulse wave data.
FIG. 14(a) is a flowchart for the enrollment mode for the embodiment in FIG. 13.
FIG. 14(b) is a flowchart of the operational mode for the embodiment in FIG. 13.
FIGS. 15(a) - 15(f) show examples of six distinct pulse waves illustrating the potential of pulse wave identification and identity tracking/confirmation.
FIG. 16 is a matrix illustrating the types of sensor techniques that can be employed to monitor a subject's pulse and generate different types of pulse-related data.
FIG. 17 is a graph showing the representation of two time-series (i.e., blood pressure and volume) in a two-dimensional phase space.
FIG. 18 is a diagram illustrating how a pulse wave probability distribution can be built up from pulse wave data over many wave cycles.
FIG. 19 is a diagram showing an efficient algorithm using integer operations for updating a pulse wave probability distribution.
Figure 20 is a schematic depiction of a typical spirogram resulting from forced vital capacity testing.
Figure 21 is a schematic depiction of a flow-volume loop (FVL) resulting from forced vital capacity testing.
Figure 22 is a system block diagram
Figure 23(a) is a flowchart of the enrollment mode
Figure 23(b) is a flowchart of the client identity confirmation process as part of a typical alcohol breath test.
Figure 24(a) is a flowchart of the enrollment mode for an embodiment in which the client characterization data is stored as a probability distribution.
Figure 24(b) is a flowchart of the process for client identity confirmation and updating the probability distribution.
Figure 25 depicts graphs illustrating how spirometric flow time-series data can be integrated over time to generate volume time-series data. The flow data and volume data can then be combined to generate a FVL.
Figure 26 is a diagram illustrating the manner in which a probability distribution can be built up from spirometric data over many respiratory cycles.
Figure 27 is a cross-sectional view of a breath alcohol testing device in its initial locked state.
Figure 28 is a cross-sectional view of the breath alcohol testing device from figure 27 in its activated state at the beginning a breath alcohol test.
Figure 29 is a cross-sectional view of the breath alcohol testing device from figures 27 -28 at the end of the breath sample.
Figure 30 is a cross-sectional view of the breath alcohol testing device from figures 27 -29 illustrating gas flow from the sample chamber through the small holes in the diaphragm into the fuel cell.

### DETAILED DESCRIPTION OF THE INVENTION

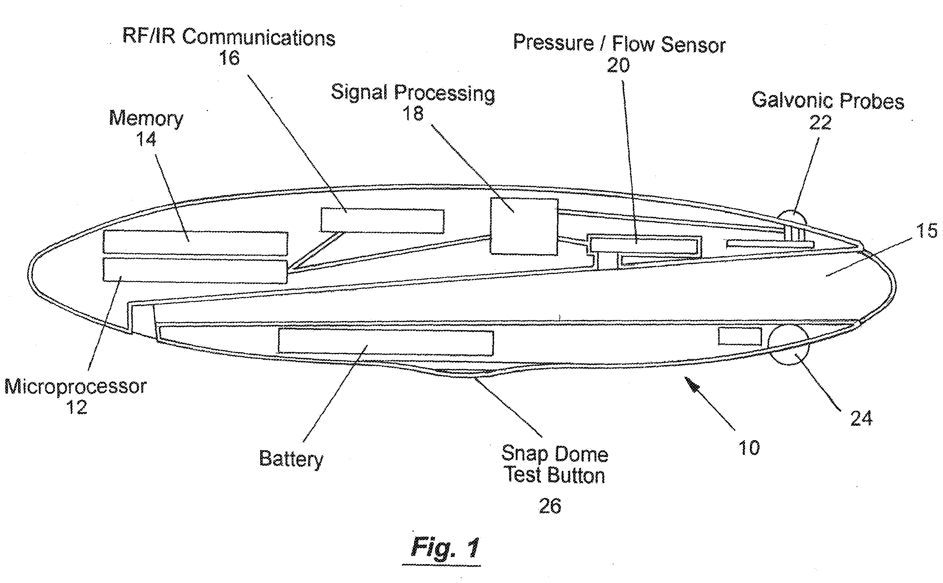

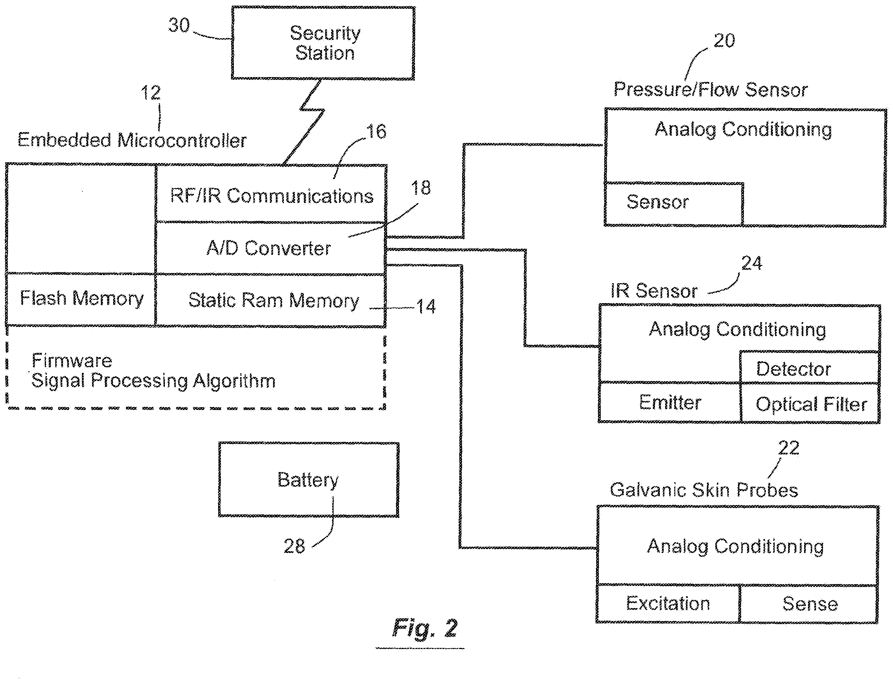

**Portable Testing Unit.** Turning to FIG. 1, a simplified cross-sectional view is illustrated FIG. 2 is a corresponding system block diagram of the portable testing unit 10. This portable testing unit 10 is designed to confirm an individual's identity through the simultaneous measurement of their exhaled breath characteristics and their pulse waveform characteristics. When paired via a communications link to an external security station 30, the portable testing unit 10 can be employed to provide secure access of any type including facilities, cars, electronic devices and secure financial transactions.

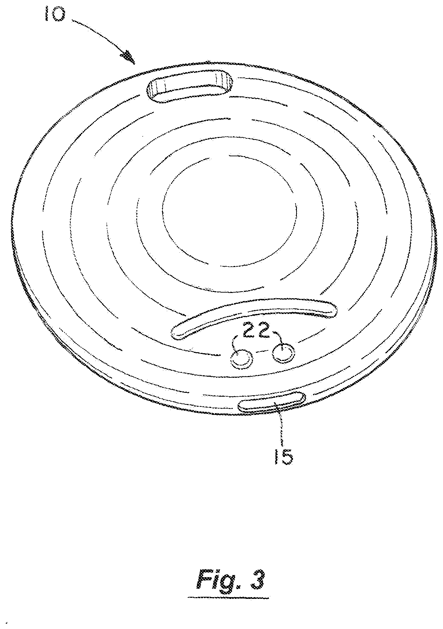

FIG. 3 is a top perspective view of an embodiment of the portable testing unit 10 with a disc-shaped housing having a diameter of about 1.75 inch. The testing unit 10 contains a pressure/flow transducer 20 within an orifice 15 extending through the testing unit 10 for generating spirometric data during breath samples provided by the subject. A pulse sensor 24 (e.g., a 910 nm IR emitter and detector 24 with two ball lenses) is located adjacent to the orifice 15 to generate pulse waveform data simultaneous with the spirometric data during each test. Two galvanic probes 22 ensure that subject's lips remain in contact with the orifice 15 throughout the test. The test unit 10 also includes a microprocessor 12 with on-board memory 14, and an analog-to-digital converter 18 as an interface between the sensors 20, 24 and the processor 12. An R/F transceiver 16 enables the testing unit 10 to communicate with an external security station 30. Finally, the testing unit 10 includes a snap dome switch 26 to initiate a test, and a battery 28 to power the remaining components.

The testing unit 10 is initially assigned by a supervising party to an individual wishing to access secure areas/systems/devices that the testing unit 10 is paired with. At this time the individual will be required to complete an enrollment process under supervision of the assigning party. During the enrollment process, the subject is required to provide a number of breath samples that enable the processor 12 to generate subject characterization data derived from the spirometric data and pulse wave data to identify the known subject. This subject characterization data is stored in the memory 14 for later use. In the subsequent operational mode, the testing unit 10 is used to confirm the person's identity through analysis of spirometric data and pulse wave data from subsequent breath samples.

In the preferred embodiment of the present invention, the testing unit 10 simultaneously performs breath print spirometry and lip pulse photoplethysmography. However, any of a variety of techniques for breath print spirometry for client identity confirmation during breath alcohol microsampling that can also be employed as will be discussed below. In the preferred embodiment of the present invention, the spirometry sensor 20 uses a pressure-sensitive diaphragm to infer the exhaled volumetric airflow rate, which can be characterized by the duration of exhalation (T), the force vital capacity (V), and the normalized shape of the flow versus time curve (S).

Providing repeatable spirometric data requires the subject to exert labial pressure on the mouthpiece or orifice 15 of the testing unit 10 to ensure a good seal. This affords the opportunity to perform simultaneous lip pulse photoplethysmography of the pulse wave using, for example, an infrared light-emitting diode (IR LED) and detector built into the housing of the testing unit 10 adjacent to the orifice 15 and in contact with subject's lip. Several approaches to using the pulse waveform for subject identity confirmation are discussed below and can be readily adopted in the present invention. For example, subject pulse waveform characteristics such as rate, excursion and shape can be measured before, during and just after exhalation. Several aspects of the pulse waveform and its interaction with the spirometric data may be exploited, such as: (1) The baseline pulse waveform itself, before exhalation; (2) The degree of exsanguination of the labial tissue as pressure is applied to achieve a seal prior to exhalation; (3) The possible acceleration and lessening excursion of the pulse as exhalation proceed; or (4) The return to normal sanguinity, rate, and excursion after exhalation has ended. The resulting combination of involuntary autonomic and physiological characteristics, subconscious idiosyncrasies and deliberate practices are believed to be quite specific to the subject, thereby providing a powerful identity authentication technique.

The following is a description of the operational mode of the present invention. When an individual wishes to access a secure system/device/area, the subject simply presses the test button 26 and places the inlet orifice 15 to their mouth with their upper lip on the galvanic probes 22 and lower lip on the IR ball lens of the pulse sensor 24. The subject then exhales completely into the device. Simultaneously, the IR emitter of the pulse sensor 24 transmits light at a wavelength of about 910 nm into the person's lower lip through ball lens. The IR detector or photodiode of the pulse sensor 24 measures IR light that has traveled through the lip and entered the ball lens. Simultaneously, the breath sample travels through the inlet orifice 15 to a flow restriction in the exit port generating a positive pressure monitored by the spirometric sensor 20. The galvanic probes 22 act as a switch to initiate the IR data acquisition and as a safety device to shut off the IR emitter and fail the test in the event a person's lip is removed during the testing period. During this period, the testing unit 10 simultaneously measures exhaled breath with the spirometric sensor 20 and pulse characteristics with the pulse sensor 24. This data is sent from the spirometric transducer 20 and pulse sensor 24 to the A/D converter 18 and is stored in memory 14 for subsequent processing by the microprocessor 12. These data are compared with the original subject characterization data from the enrollment period to confirm the subject's identity. In the event the identity is not confirmed, an encrypted lockout code is transmitted along with the testing unit's unique identifier code. In the event the individual's identity is confirmed the testing unit 10 transmits a unique encrypted identifier and an enable code to the security station 30 for a predetermined time frame. Access devices that have been paired with this unique testing unit 10 will provide or deny access based on the identity confirmation test results.

The present disclosure provides a number of key advantages over the prior art. First, once a testing unit 10 is assigned to and enrolled by an individual, only that individual's identity can be confirmed by the testing. In the event the testing unit 10 is lost, it is useless to anyone that may find it. This is certainly not the case with a traditional office key, car key, house key, RFID key, swipe access card or credit/debit card.

Second, due to the testing unit's ability to complete an identity confirmation test in advance of entering the facility and transmit an enable access code for a period of time, it can dramatically reduce waiting time for employees entering secure facilities, in contrast to conventional facial recognition, retina, fingerprint and voice identification methods as well as retail credit/debit card transactions. This new level of credit/debit card security is believed to have the potential to save billions of dollars annually in credit card fraud.

The same testing unit 10 may be assigned to more than one individual such as a number of members of a family. In this instance each individual would be enrolled on the testing unit 10 and would subsequently confirm the identity of each individual and record the person accessing the facility or device at that time.

Finally, the present unit may be programmed to provide different time periods of access for different levels of required security. For example, the testing unit 10 could be programmed to provide a fifteen-minute enable period for a home security system, five minutes to enable a car start, one minute for a secure office, six hours for a low level computer, and 500 milliseconds for a debit/credit/ATM card or financial transaction. It is anticipated that this device could be used by any type of secure facility, business, residence, automobile, mechanical or electronic device, debit/credit/ATM card terminal providing a convenient and enhanced level of access/transaction security.

**Method of Operation.** The following is a discussion of the preferred method of operation of the present unit. As previously discussed, the major components of the present system generally include a computer processor, data storage, a pulse sensor adjacent to the subject's tissue that generates time-series data based on the subject's pulse waves, and a spirometric sensor that measures predetermined spirometric properties of the exhaled breath sample, such as flow or pressure. This spirometric data is typically generated as time-series data over the course of the sample.

As an overview, the processor initially receives and analyzes the pulse wave data from the pulse sensor and the spirometric data from the spirometric sensor for a known subject to generate subject characterization data identifying the known subject. Thereafter, in the identity authentication mode, the processor simultaneously receives data from the pulse sensor and spirometric sensor for a test subject (who may or may not be the known subject). The processor analyzes this data in conjunction with the stored subject characterization data to determine whether the test subject is the same as the known subject. For the purposes of this application, it should be understood that the phrase "test subject" refers to the person whose identity is being tested or confirmed during the identity authentication mode of the present system.

Thus, the present system operates in one of two mutually exclusive modes - an enrollment mode and an identity authentication mode. The enrollment mode acquires subject data under the supervision of a trained technician, computes subject characteristics, calculates the probability of an impostor producing similar characteristics, and stores these findings in a client database for later use during the identity authentication mode.

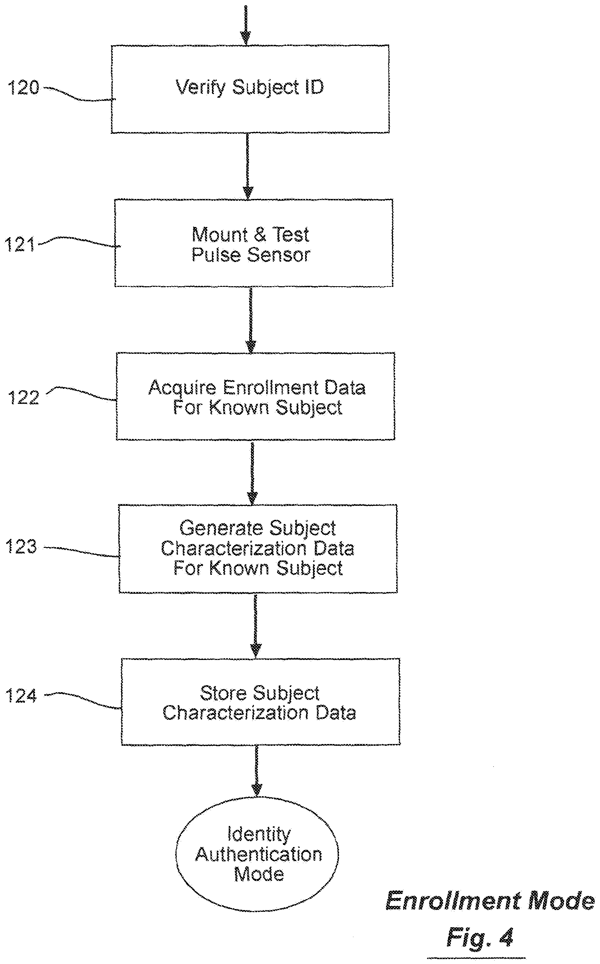

Figure 4 is a general flowchart of the enrollment mode employed to initially build subject characterization data for a known subject. The operator first verifies the identity of the subject (step 120), and mounts and tests the pulse sensor on the subject, and provides the subject with a spirometric sensor (step 121). The processor simultaneously acquires pulse wave data from the pulse sensor and spirometric data from the spirometric sensor for a brief period of time (step 122). The subject may be asked to undertake a range of activities to ensure the enrollment data are representative of that which may be encountered over the subject's normal day-to-day activities. The processor analyzes the enrollment data and generates subject characterization data for identifying the known subject (step 123). This subject characterization data is stored for later use during the identity authentication mode of the present system (step 124), as will be described below.

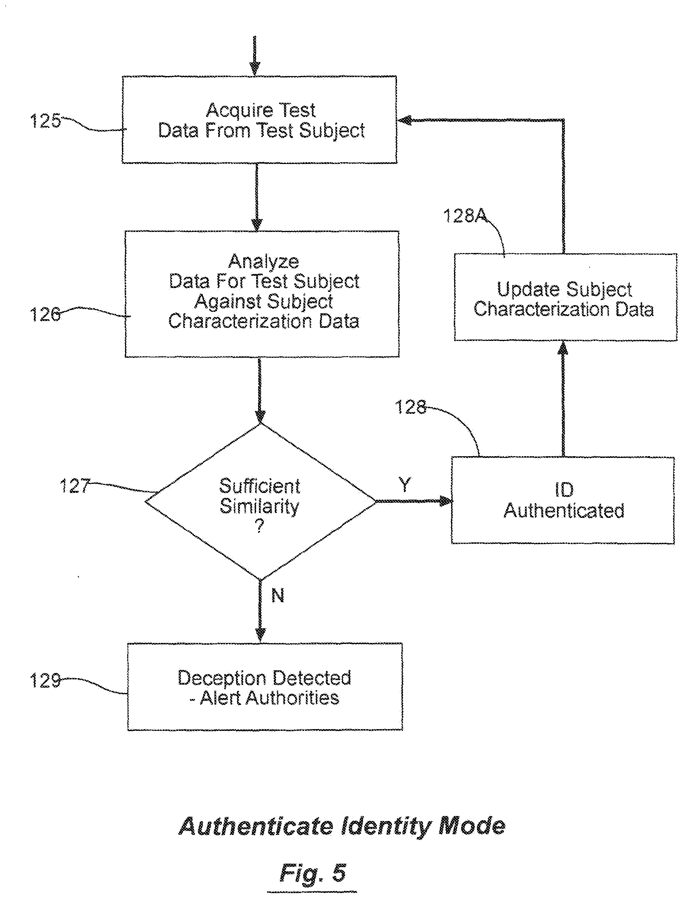

The identity authentication mode is used to authenticate the identity of a test subject, who may or may not be the known subject from the enrollment mode. In this mode, the system acquires subject data unsupervised in the field, compares it to subject and impostor characteristics, and decides whether to authenticate or challenge identification. Figure 5 is a flowchart of one possible embodiment of the identity authentication mode. For each identity authentication test, the processor acquires pulse wave data from the pulse sensor and spirometric data from the spirometric sensor for the test subject (step 125). The processor analyzes this test data using the subject characterization data (step 126). Based on this analysis, in step 127, the processor determines whether there is a sufficient degree of similarity between the pulse wave and spirometric characteristics of the known subject (from the subject characterization data) and the test subject to conclude that these subjects are the same person (step 128). If so, the processor may update the subject characterization data 118 to include the current test data (step 128A) and then loop back to step 125. Otherwise, if the processor determines that the current test subject is not the same as the known subject, an alarm can be activated to signal that deception has been detected (step 129).

As will be discussed below, the two modes in the preferred embodiment of the present invention share a common "acquire trial" procedure that acquires and pre-processes a short, contiguous time-series data of the digitized measurement, called a "trial".

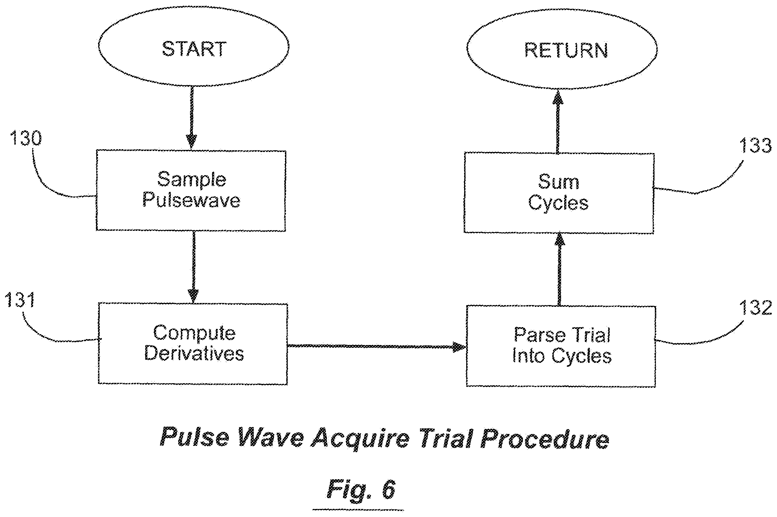

Figure 6 shows how the pulse wave algorithm acquires a trial. The trial pulse wave typically consists of about a relatively small number (e.g., ten) pulse cycles, which are similar but not identical. The goal of the procedure is to convert the multi-cycle waveform into a single representative cycle. In block 130, the processor queries the pulse sensor at intervals to sample the pulse wave and obtain digitized time-series data. In block 131, the processor calculates the first and second derivatives of the pulse wave data with respect to time, to eliminate baseline drift and generate triggers associated with the systolic excursion. Representing the subject's pulse wave with its first derivative also obscures the bio-informational nature of the signal, thus enhancing privacy. In block 132, the processor parses the trial into cycles using the second derivative to find consistent start points called "triggers" for synchronizing the cycles, based on the systolic excursion. In block 133, the processor sums corresponding points of the first derivative of the cycles, and then returns the sum cycle to the calling program.

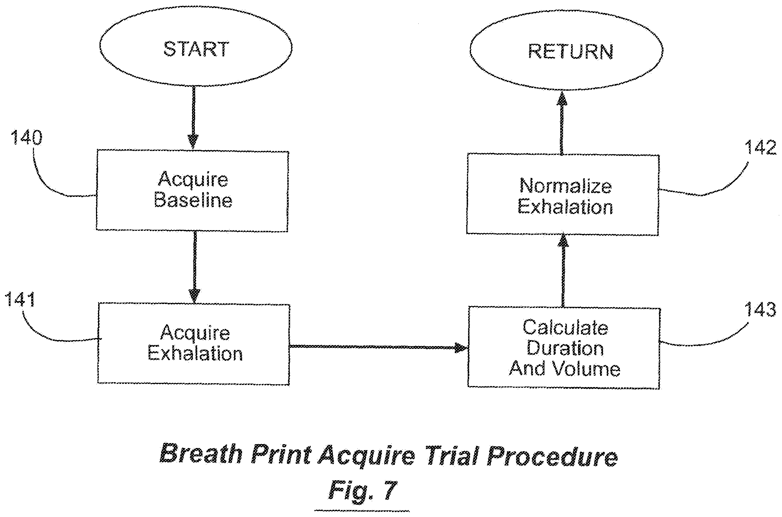

Figure 7 shows how the spirometric algorithm acquires a trial. The trial airflow consists of a single forced exhalation, preceded by a quiescent period used to establish a signal baseline, and succeeded by a period used to ensure further exhalation is not forthcoming to spoil the data. The goal of the procedure is to delineate these three periods, measure the exhalation duration and volume, subtract the baseline from the exhalation, and temporally normalize the net exhalation to its duration and volume. In block 140, the processor averages the pre-exhalation hardware signal, and watches for the onset of exhalation. In block 141, the processor records the baseline-subtracted net signal until the cessation of exhalation, and then monitors the post-exhalation for airflow resurgences. Block 143 calculates the duration and volume of the exhalation period. Both duration and volume can be used as identifying characteristics in their own right, and to normalize the exhalation airflow shape as another identifier. The net exhalation data is normalized in block 142 by compressing this data set into a standard length and volume for comparison to other spirometric trials.

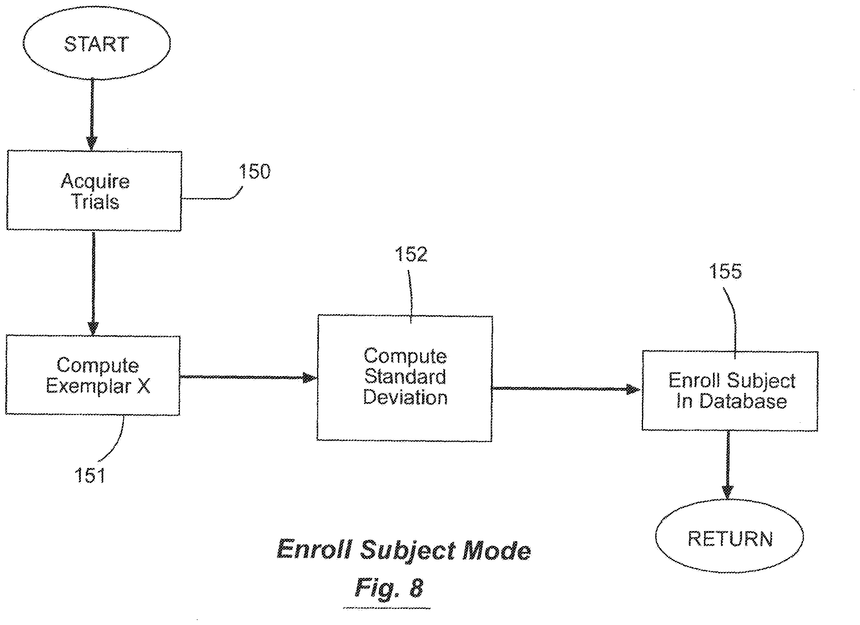

Figure 8 shows a first embodiment of the procedure used by both the pulse wave and spirometric algorithms to enroll a new client. This procedure can be use both to establish the individual's characteristics as a subject whose identity will be putative in the field, and as a possible impostor for any other client. Block 150 calls the "acquire trial" procedure (discussed above) at the start of each enrollment trial. In block 151, the processor computes an exemplar (X) by calculating the normalized pulse wave vector in the case of the pulse wave algorithm, the mean exhalation duration and volume scalars and normalized airflow shape vector in the case of the spirometric algorithm, or both of these in the combined algorithm. In block 152 ("Compute Standard Deviation"), the processor calculates the scalar pulse wave variation in the case of the pulse wave algorithm, the duration, volume and shape variation scalars in the case of the spirometric algorithm, or both of these in the combined algorithm. The new information for the subject can be stored in a database for future use in block 155.

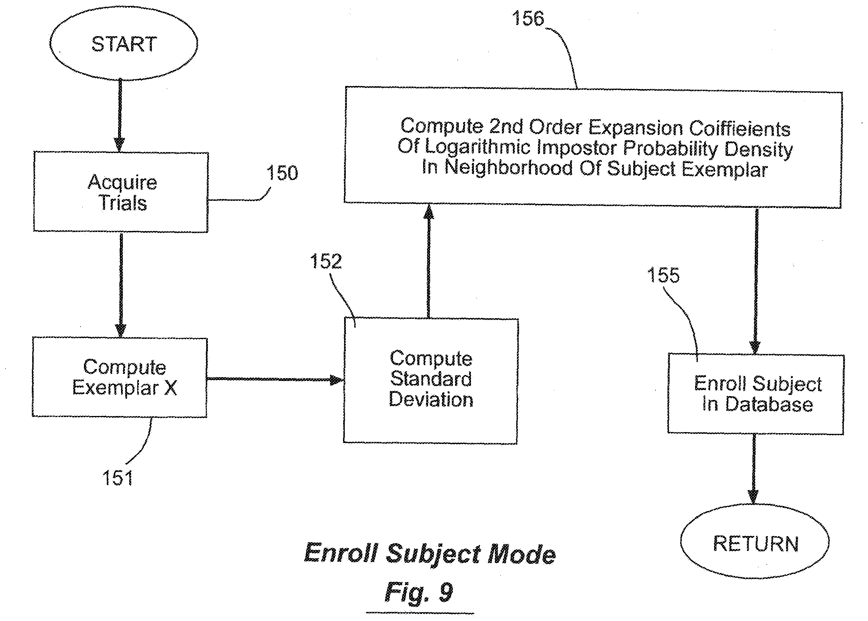

Figure 9 is flowchart showing a second embodiment of the enrollment procedure in which blocks 153 and 154 in Figure 8 have been replaced in block 156 with a computational shortcut or approximation. Here, second-order expansion coefficients of the logarithmic impostor probability density in the neighborhood of the subject exemplar are computed and stored. This approximate algorithm allows an arbitrarily large imposter database to be stored in a compact, but approximated form on the sensor, and spares the sensor from the calculation of special mathematical functions. In contrast, the more exact algorithm in Figure 8 makes no approximations and uses actual impostor exemplars stored in the sensor for subsequently computing the impostor probability density at the field trial. However, this limits the impostor database size and requires the sensor to calculate special functions.

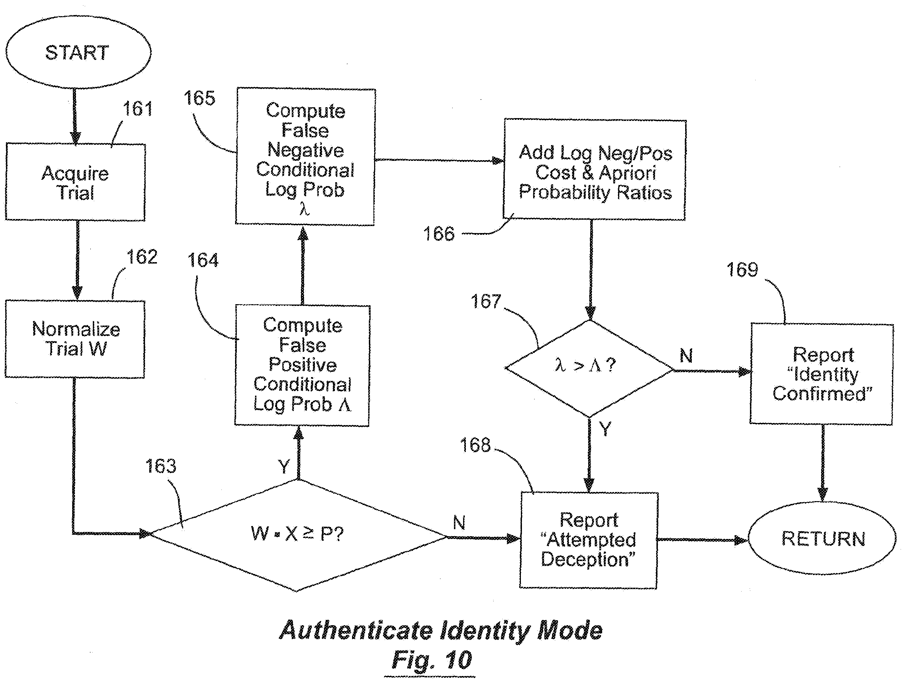

Figure 10 shows how either of the algorithms decides whether it is better to authenticate or challenge the subject's identity, based on a field trial. The first two blocks 161, 162 respectively acquire and normalize a field trial (W). The processor calculates the dot product of the normalized field trial (W) and subject's exemplar (X), and compares it to a preset proximity threshold P (block 163). If W•X is smaller than P, the algorithm reports "attempted deception" (block 168), if not, processing continues with block 164. In block 164 ("Compute False Positive Conditional Log Prob. Λ"), the processor assesses the likelihood of measuring the actual field trial, given the subject as donor. Here, a false positive is reporting "attempted deception", when the test subject is in fact the known subject. The logarithm of the subject probability density at the field trial is Λ.

In block 165 ("Compute False Negative Conditional Log Prob. λ"), the processor also assesses the likelihood of measuring the actual field trial, given a typical impostor as donor. In other words, a false negative is reporting "identity confirmed", when an impostor is in fact the test subject. The logarithm of the imposter probability density at the field trial is λ.

The processor then makes a Bayesian assessment of probabilities. In block 166 ("Add Log Neg/Pos Cost & A Priori Probability Ratios"), the processor adjusts λ for the relative penalty of each type of false decision, and a beforehand estimate of the particular subject's probability of attempting deception., The processor then chooses the larger of λ and Λ in block 167 ("λ > Λ?"). If λ is larger, the processor reports attempted deception (block 168). If Λ is larger, the processor reports "identity confirmed" (block 169).

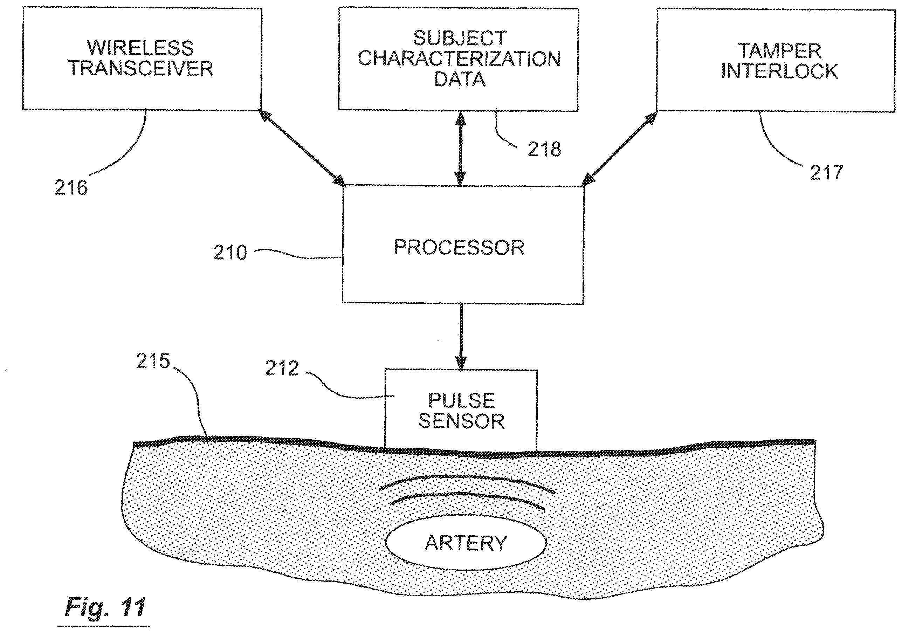

**Biometric Identity Confirmation System Using Pulse Wave Data.** Figures 11 - 19 illustrate an unclaimed alternative embodiment of the present disclosure that characterizes individuals by the non-invasive sensing of arterial pulse waves, for the purpose of identification and identity tracking/confirmation. The previous embodiment used the combination of spirometric data and pulse wave data, which provides a significantly higher degree of confidence in subject identification and identity tracking/confirmation. Although providing a somewhat lower degree of confidence, a biometric identity confirmation system based on characterization of pulse wave data (without spirometric data) may be good enough in some fields of use, particularly where lower cost and simplicity of design are significant concerns.

FIG. 11 is a simplified system diagram for this embodiment. The major components include a computer processor 210, and a pulse sensor 212 adjacent to the subject's tissue 215 that generates time-series data based on the subject's pulse waves.

As an overview, the processor 210 initially receives and analyzes this pulse wave data from the pulse sensor 212 for a known subject to generate subject characterization data 218 identifying the known subject. Thereafter, in normal operational mode, the processor 210 receives pulse wave data from the pulse sensor 212 for a test subject (who may or may not be the known subject). The processor 210 analyzes this pulse wave data in conjunction with the subject characterization data 218 to determine whether the test subject is the same as the known subject. For the purposes of this application, it should be understood that the phrase "test subject" refers to the person whose identity is being tested or confirmed during the operational mode of the present system.

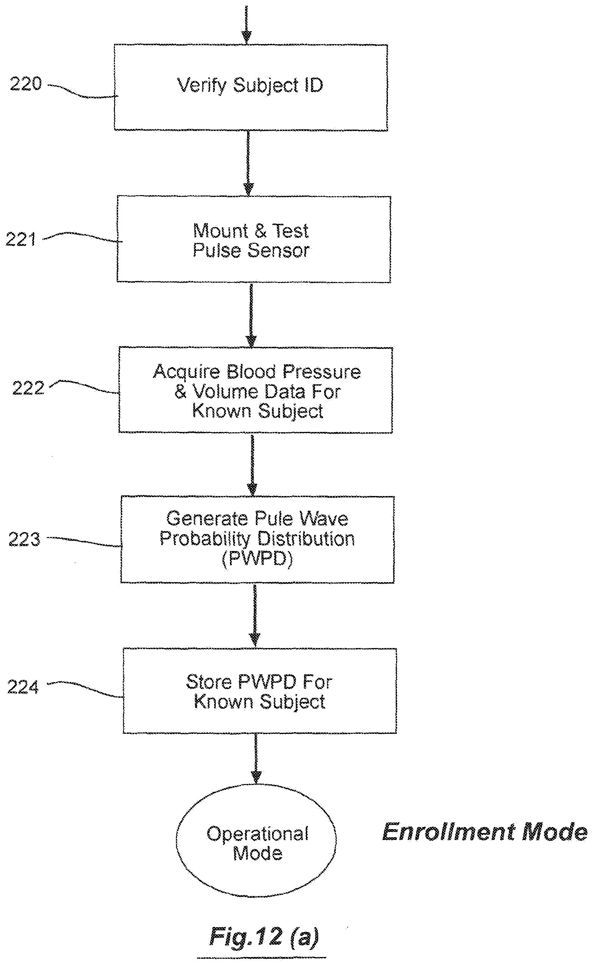

FIG. 12(a) is a flowchart of the enrollment mode employed to initially build subject characterization data 218 for a known subject. The operator first verifies the identity of the subject (step 220), and mounts and tests the pulse sensor 212 on the subject (step 221). The processor 210 acquires pulse wave data from the pulse sensor 212 for a brief period of time (step 222). The subject may be asked to undertake a range of activities to ensure the enrollment pulse wave data is representative of that which may be encountered over the subject's normal day-to-day activities. The processor 210 analyzes the enrollment pulse wave data and generates subject characterization data 218 for identifying the known subject (step 223). This subject characterization data 218 is stored for later use during the operational mode of the present system (step 224) as will be described below.

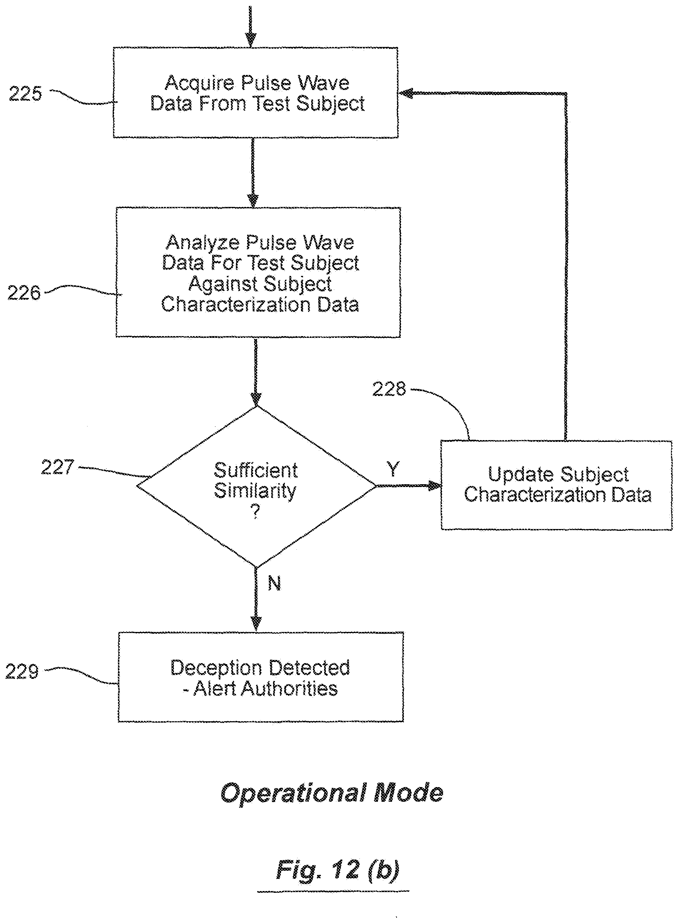

Following completion of the enrollment mode, the present system proceeds to operational mode during day-to-day monitoring of the subject. FIG. 12(b) is a flowchart of the operational mode. At selected time intervals or on a continuing basis, the processor 210 acquires pulse wave data from the pulse sensor 212 for the test subject (step 225). The processor 210 analyzes this pulse wave data using the subject characterization data 218 (step 226). Based on this analysis, the processor determines whether there is a sufficient degree of similarity between the pulse wave characteristics of the known subject (from the subject characterization data 218) and the test subject to conclude that these subjects are the same person (step 227). If so, the processor 210 may update the subject characterization data 218 to include the current pulse wave data (step 228) and then loop back to step 225. Otherwise, if the processor 210 determines that the current test subject is not the same as the known subject, an alarm can be activated to signal that deception has been detected (step 229). The processor can also remotely alert the authorities via a wireless transceiver 216.

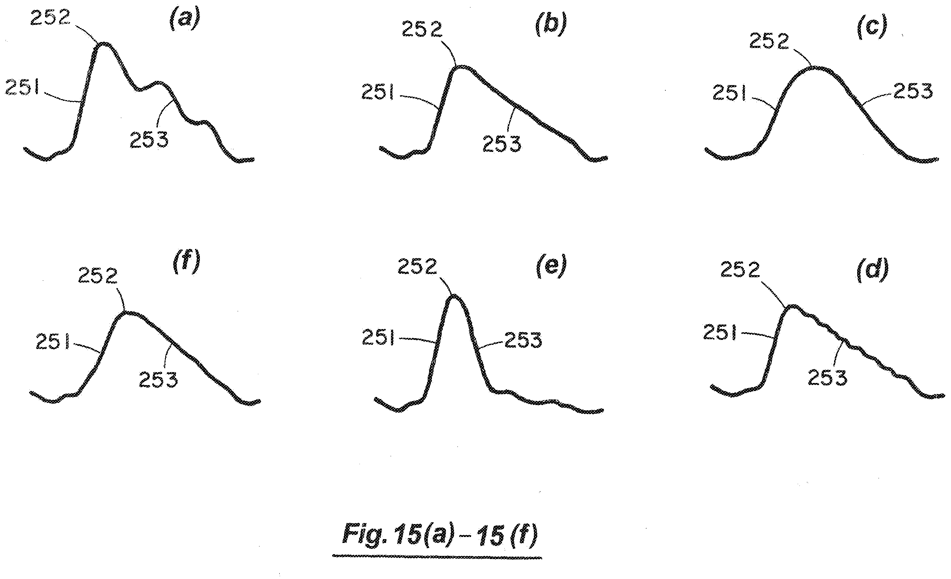

FIGS. 15(a) - 15(f) show examples of six distinct pulse waves. Qualitative characteristics useful for identification include the abruptness of systolic onset (leading edge), the roundedness of systole (peak), the concavity of diastolic onset (trailing edge), the presence or absence of the dichrotic notch (dip) and other oscillations, and the timing of oscillatory features relative to systole. In contrast to the three most common hemodynamic measurements - systolic pressure, diastolic pressure, and pulse rate - these characteristics are persistent through cycles of sleep and waking, leisure and exertion, and relaxation and stress. Pulse wave characteristics do evolve as the subject ages, but these changes are negligible over the identity-tracking/confirmation time scale.

The leading edge 251 of the pulse wave in FIG. 15(a) has a fast slope. The peak 252 of the pulse wave has a rounded, but narrow crest that is not delayed. The trailing edge 253 of the pulse wave exhibits dicrotism. In contrast, the trailing edge 253 of the pulse wave in FIG. 15(b) has no dicrotism. The peak 252 of the pulse wave in FIG. 15(c) is delayed and has a round crest. The pulse wave in FIG. 15(d) shows a pulse wave with a trailing edge 253 having a series of small waves. FIG. 15(e) shows a pulse wave with a trailing edge 253 that exhibits deep dicrotism. FIG. 15(f) has a leading edge 251 with a slower slope, a peak 252 that is delayed but not rounded, and a trailing edge 253 with no dicrotism.

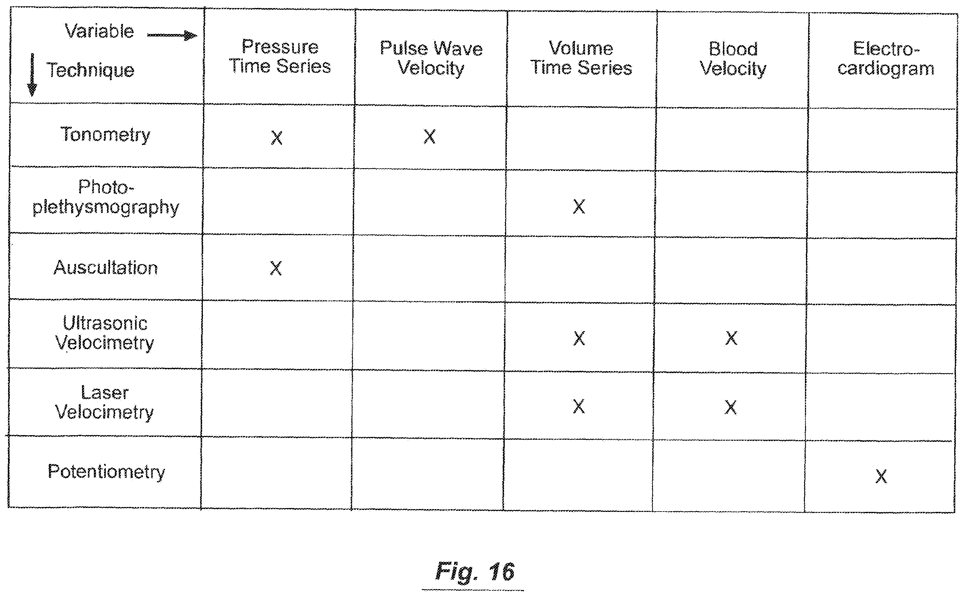

FIG. 16 is a matrix illustrating the types of measurement techniques that can be employed to monitor a subject's pulse and generate different types of pulse-related data. Possible arterial blood transport measurements include the blood pressure time-series, the pulse wave velocity, the blood volume time-series, and blood velocity. The electrocardiogram (EKG), although not a transport measurement per se, should also be considered due to its own potential for identification, and as a master timer for synchronous detection.

Pressure and volume time-series are local measurements in the sense of requiring but a single bodily contact, but are global in the sense that the heart and remote features of the arterial system influence the measurement through forcing, viscous drag, and pressure wave reflections. Thus the entire subject may be characterized using a point sensor, as with established biometric identification techniques such as fingerprint and retinal scans.

Identification and tracking/confirmation should be non-invasive (i.e., the pulse sensor 212 should contact but not penetrate the subject). All of the variables listed above can be measured non-invasively by the pulse sensor 212, using techniques such as tonometry, photoplethysmography, auscultation, ultrasonic Doppler flowmetry, laser Doppler flowmetry and potentiometry.

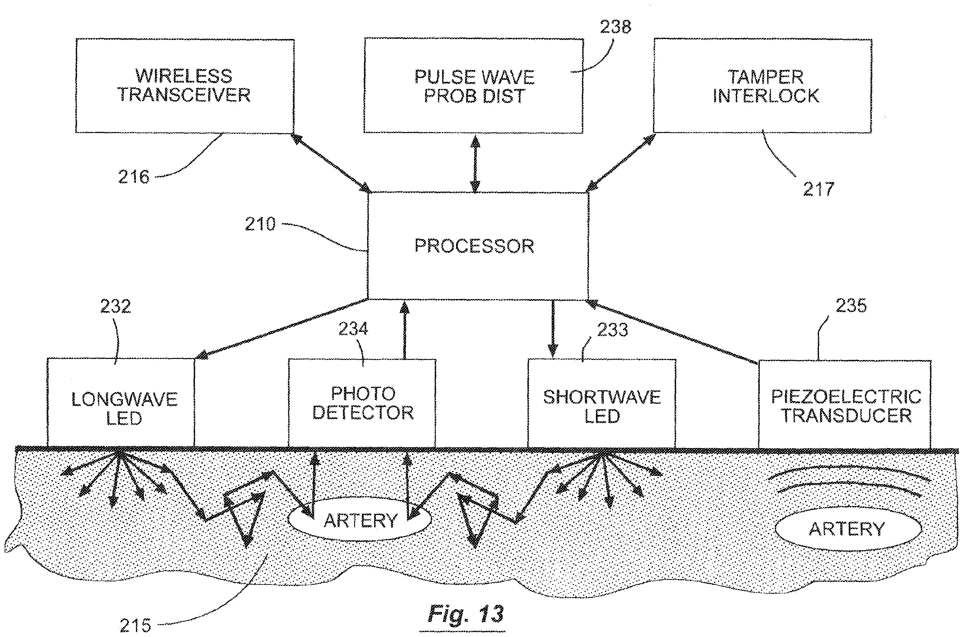

Tonometry and photoplethysmography appear to be the two most promising pulse-wave sensing techniques for subject identification, and will serve to illustrate the disclosure. A piezoelectric transducer can perform tonometry, as shown in FIG. 13, and is core to the compact commercial instrument "PulsePen" marketed by DiaTecne s.r.l. of Milan, Italy. Two light-emitting diodes 232, 233 and a photodetector 234 can perform photoplethysmography as shown in FIG. 13, the dominant technology in the pulse-oximeter market.

Simultaneous tonometry and photoplethysmography may be particularly effective. The relative magnitude of the pressure and volume swings is a measure of arterial elasticity, and the phase lag from the pressure to the volume peak gives a hemodynamic parameter called the Womersley number.

Pressure and volume time-series are attractive hemodynamic variables, since each provides non-local information from a single-contact sensor. These may be implemented by tonometry and photoplethysmography, respectively. Both technologies are simple, low-power, and mature bases of commercial sensors.

Thus, the present disclosure can be based on any of the more promising hemodynamic variables, sensing techniques, and signal processing algorithms. This example is illustrative only, and should not be construed as our relinquishment of the alternative variables, techniques, and algorithms discussed here, or uncovered later, for the purposes of pulse wave identification and identity tracking/confirmation. In particular, a wide variety of signal processing techniques in either the time domain or frequency domain can be applied to the pulse wave data output by the pulse sensor 212 to generate subject characterization data 218.

Subject characterization can be approached in any of several ways: (1) A classification according to qualitative features of the pulse wave, such as the relative timing of the systolic peak and the inflection point; (2) A local scalar parameter, such as the arterial elasticity or Womersley number; (3) A non-local scalar parameter, such as the time delay between forward and reflected pressure waves, which is equal to the distance to the reflecting structure divided by the pulse wave velocity; (4) A vector parameter, such as that resulting from fitting a Windkessel RCL-network model (see, for example, "Arterial pressure contour analysis for estimating human vascular properties", T. B. Watt et. al., Journal of Applied Physiology 40, pp. 171-176 (1976)); or (5) A learned probability density in phase space, such as the delayed correlation of either the pressure or the volume with itself, or the simultaneous correlation of pressure and volume. The qualitative approach has a parallel in fingerprint analysis, in which the Henry system of classification uses loops, whorls, and arches to sort fingerprints. While qualitative classification undoubtedly helpful in forensics and cardiology, it has shortcomings that may limit its appropriateness for this disclosure. To suffice in itself, a scalar parameter needs a spread among individuals of a population that is large compared to the variation of a particular individual from one occasion to another. That any single pulse wave parameter fits the bill is dubious: The population spread of pulse wave parameters is not dissimilar to that of adult height, and while height is useful in identification, few would assert that height alone is sufficient.

A vector parameter may be better suited for subject identification, owing to its multiple dimensionality. A Windkessel fit has demonstrated pairs of individuals distinguishable from each other by their arterial compliance (C), but not by their viscous resistance (R), and vice versa ("Identification of vascular parameters based on the same pressure pulses [sic] waves used to measure pulse wave velocity", A. S. Ferreira et. al., 23rd Annual International Conference of the IEEE Engineering in Medicine and Biology Society (2001)). However, the circulatory system is not a passive electrical circuit, being both non-linear and non-local; and poorly fitting models tend to yield unstable parameter values.

The learned-probability approach recommends itself in several ways: (1) It can't bomb out because of an individual's lack of a common but non-universal hemodynamic feature, such as the dichrotic notch; (2) It utilizes all data, rather than heavily weighting prominent features, such as the systolic peak; (3) It relies on no artificial or simplistic assumptions about the dynamics, as does the Windkessel approach; and (4) It naturally yields the optimal decision and probability of error in detecting identity deception. Therefore, the learned-probability approach will serve to illustrate the example. In the present context, "phase space" is a multi-dimensional (D-dimensional) space in which a quasi-periodic variable is correlated with (D-1) other measurements. The other measurements can be the same variable measured at various times in the past, or other contemporary variables, or a combination. The D measurements form a vector that traces an "orbit" in phase space. A strictly periodic phenomenon will follow the same orbit over and over, and will soon be utterly predictable. A phenomenon that varies from cycle to cycle will yield a blurred, probabilistic orbit.

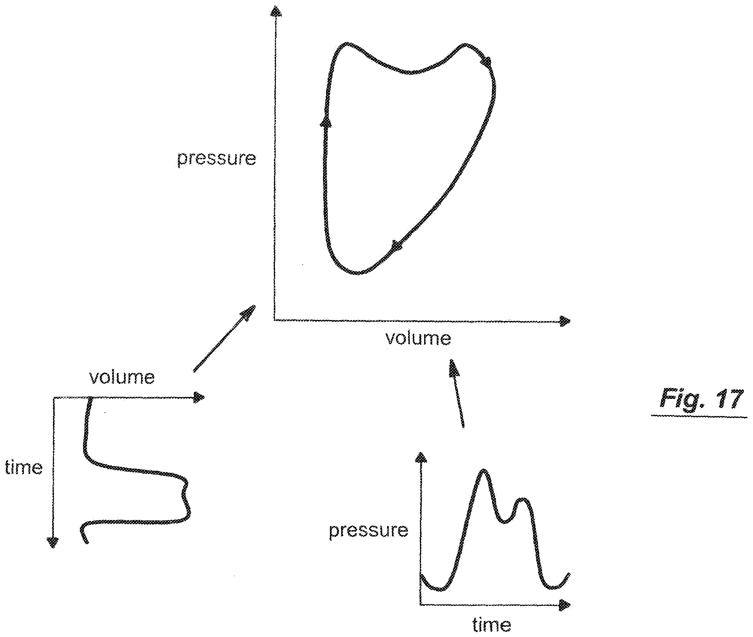

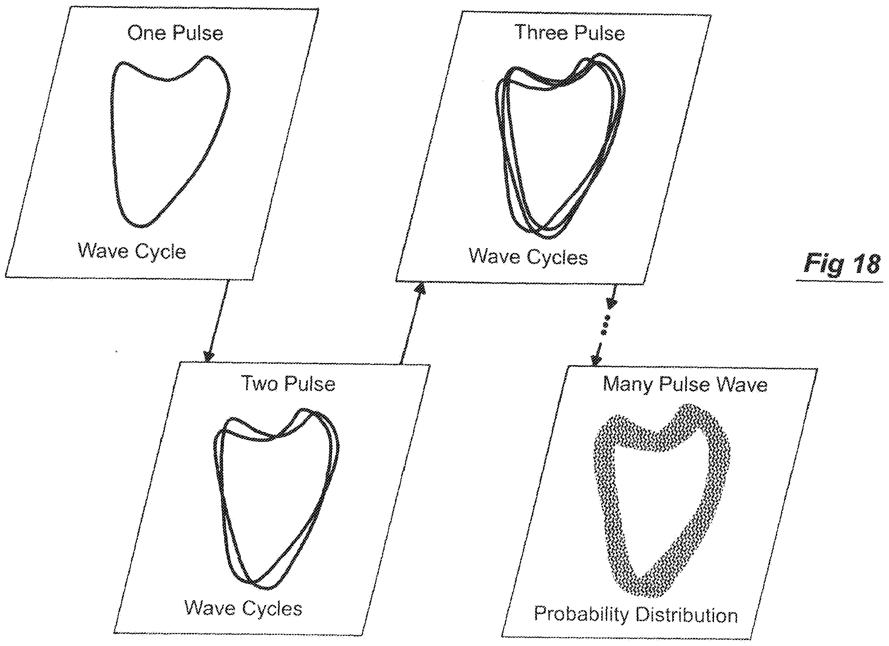

It happens that blood volume lags blood pressure by a fraction of a cycle, so a reasonable choice is the 2-D phase space comprising the present values of pressure and volume. FIG. 17 depicts the reduction of pressure and volume time-series into a pressure versus volume orbit in phase space. FIG. 18 is a diagram illustrating how a pulse wave probability distribution can be built up from pulse wave data over many wave cycles.

The phase space domain is usually just the outer product of its scalar variable domains. With 8-bit analog-to-digital conversion (ADC), a 2-D phase space needs but a modest 65,536-address memory. An 8-bit ADC is probably adequate, considering the small dynamic range and noisiness of the signals. An example pressure (volume) domain is 64 mmHg to 192 mmHg, in 0.5 mmHg steps (1/2 to 3/2 the average volume, in steps of 1/256).

The phase space range should be appropriate for storing a probability - for instance, an unsigned integer. Since the orbit visits some phase space cells much more frequently than others, the integer must have sufficient dynamic range, say 16 bits. Thus, the example phase space probability density memory requirement is 128 kilobytes. In other words, the phase space is effectively a 2-D array of cells or elements, each of which store an integer value representing the probability associated with a particular pair of a blood pressure and volume values for the subject. This phase space can be referred to as a pulse wave probability distribution (or PWPD)

FIG. 13 is a system diagram of an embodiment using blood pressure and pulsatile blood volume as the pulse wave data used for generating a pulse wave probability density 238 for the purpose of subject characterization. The embodiment illustrated in FIG. 13 also employs a photoplethysmograph as the pulse wave sensor. Two light-emitting diodes 232, 233 irradiate the subcutaneous tissue 215, and a common photodetector 234 senses the backscattered light. One LED 232 emits at a longer wavelength whose absorption is dominated by oxygenated hemoglobin, and the other LED 233 emits at a shorter wavelength whose absorption is dominated by deoxygenated hemoglobin. The LEDs 232, 233 can be modulated out of phase to temporally multiplex their signals, and the pulsatile blood volume is proportional to the difference signal.

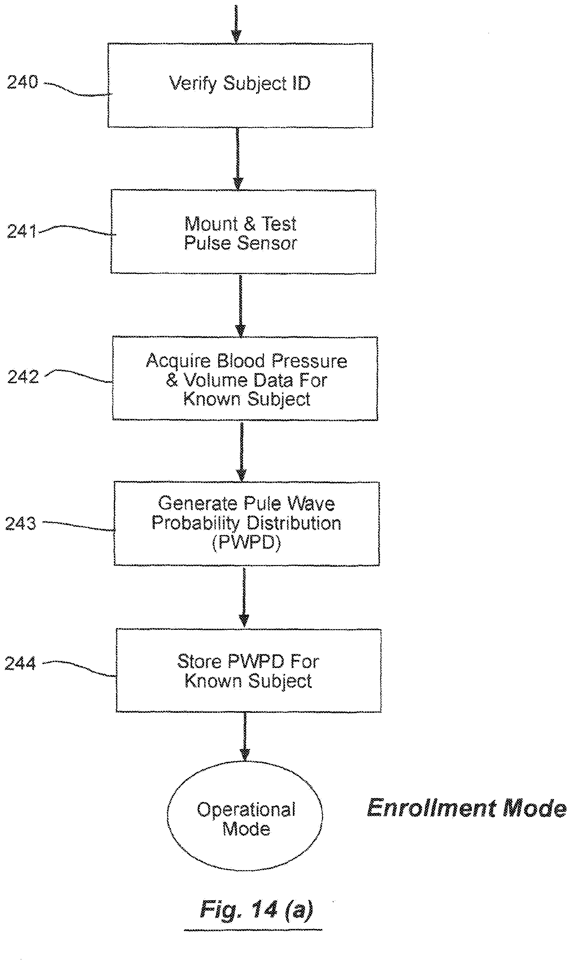

A known subject's pulse wave probability density 238 is initially acquired during the brief enrollment period, consisting of the subject wearing the pulse sensor 212 while engaging in various activities. FIG. 14(a) is a flowchart for this enrollment mode for the embodiment illustrated in FIG. 13. Here again, the operator verifies the identity of the subject (step 240), and mounts and tests the LEDs 232, 233 and photodetector 234 on the subject (step 241). The processor 210 acquires blood pressure and volume data from the photodetector 234 for a brief period of time (step 242). The processor analyzes this data to generate a pulse wave probability distribution (PWPD) 238 for the known subject in step 243. In particular, the pulse wave probability density 238 can be generated from blood pressure time-series data correlated with blood volume time-series data. The PWPD 238 is then stored for later use in the operational mode (step 244).

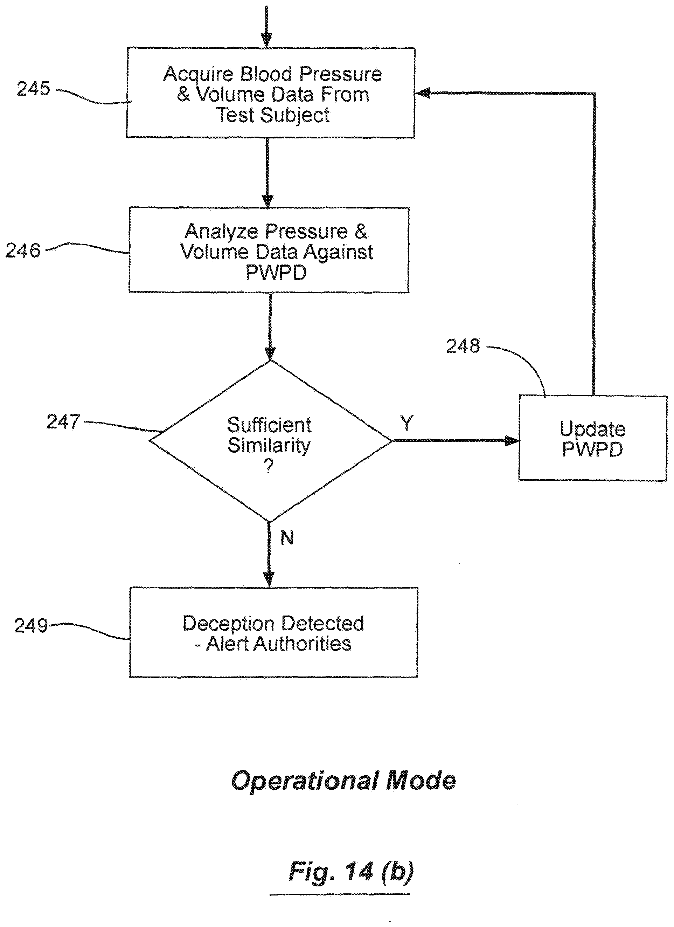

After enrollment, the present monitoring system moves to operational mode. FIG. 14(b) is a flowchart of the operational mode for the embodiment of the present invention shown in FIG. 13. During each iteration, the processor 210 acquires blood pressure and volume data from the photodetector 234 for the test subject (step 245). The processor 210 analyzes this blood pressure and volume data to determine there is sufficient similarity between the pulse wave characteristics of the known subject and the test subject currently wearing the present unit (steps 246 and 247).

More specifically, the pulse wave probability distribution 238 serves as a look-up table for the probability associated with pairs of blood pressure and volume values measured during operational mode. In particular, the processor 210 retrieves from the pulse wave probability distribution 238 the probability associated with the current blood pressure and volume values. While not perfectly predictive, the pulse wave probability density 238 contains a great deal of information about the relationship of different phases of the cycle to each other, and can be quite specific to a subject, without assuming any particular model.

In the preferred embodiment of the present invention, deception is detected when the compound probability of measuring the latest N data is deemed sufficiently small. More specifically, a deception is judged when the cost of erroneously regarding the subject as truthful exceeds the cost of erroneously regarding the subject as deceptive: C(t|D)×P(D|M) > C(d|T)×P(T|M), where C(t|D) is the penalty for judging the subject truthful when in fact deceptive, and P(D|M) is the (unknown) conditional probability of deception given the measurement M, and vice versa for the right-hand side of the inequality.

Bayes' theorem states P(D|M)×P(M) = P(D,M) = P(M|D)×P(D), where P(M) is the (inconsequential) a priori probability of measuring M, P(D,M) is the (undesired) joint probability of deception and measuring M, P(M|D) is the (known) conditional probability of measuring M given deception, and P(D) is the (estimated) probability of deception. Substituting into the cost condition and rearranging gives P(M|D)/P(M|T) × P(D)/P(T) > C(d|T)/C(t|D). These factors are all known or estimated: P(M|D) is given by the average of all subjects' probability densities, assuming this average represents the general population, and the subject is as likely to pass off the sensor to anyone as to anyone else; P(M|T) is given by the subject's own probability density; P(D) is estimated from a subject's past behavior (e.g. a subject who has not attempted deception in a year has at most a 10⁻⁶ probability of attempting deception in any 30-second measurement period); P(T) is merely 1-P(D); and C(d|T) and C(t|D) are input parameters.

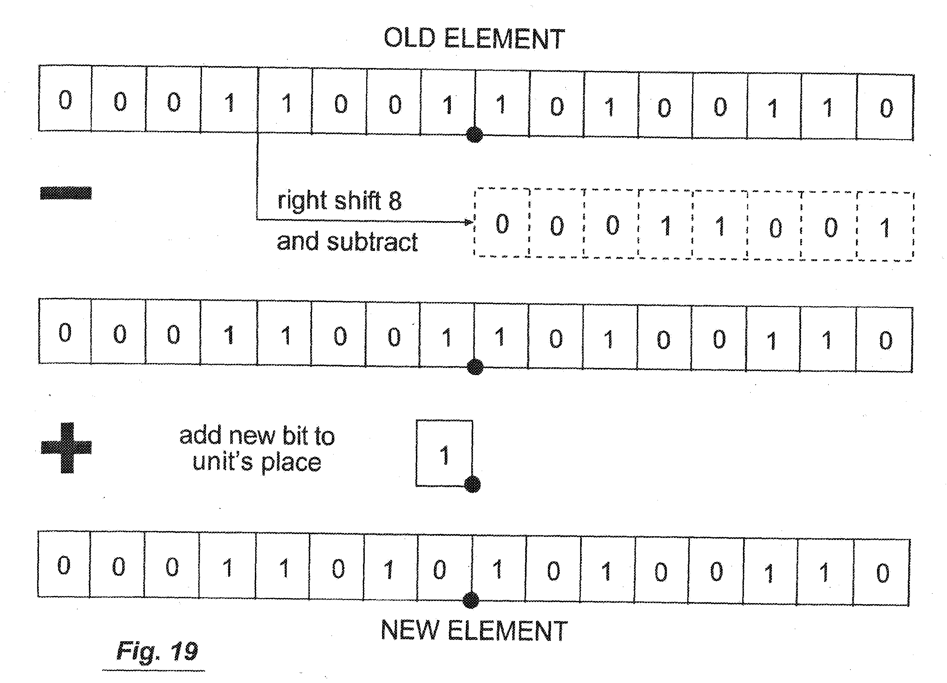

If the processor 210 determines deception has occurred, an alarm can be activated and the authorities are alerted (step 249). Otherwise, before returning to step 245 to begin the next iteration, the blood pressure and volume data from this iteration are employed to update the pulse wave probability density 238 (step 248). In order to weight new data more than old data, and to prevent overflow, the accumulated probability density is continuously devalued. For example, the pulse wave probability density 238 can be efficiently updated in step 248 of FIG. 14(b) using the elementary operations illustrated in FIG. 19. After each measurement, and for each element in the phase space, devalue the existing probability density (e.g., by multiplying by 255/256) to account for the decay of information's relevance over time, then add a new data bit (e.g., 1) to the unit's place. The actual probability can be normalized to the sum of elements over the space, of course, but this conventional normalization is not needed in the following algorithm, saving computations.

The present disclosure can be employed in a number of possible fields of use. For example, it can be used as a self-contained, mobile unit for identity confirmation as part of an alcohol monitoring system, such as an alcohol monitoring bracelet or a vehicle interlock system to prevent operation by an unauthorized or alcohol-impaired driver. The present disclosure can also be used to remotely track and verify the identity of persons at a secure facility or under house arrest. For example, this identity verification can be performed continually, at selected time intervals, or at selected locations in the facility.

Presently, many automated identity confirmation systems in secure facilities read a magnetic stripe or barcode on badges, or rely on biometrics such as fingerprint or retinal scans. The drawbacks to existing approaches include: (1) Badge-based identity confirmation is easily subverted, providing security too weak for many applications; (2) Biometric approaches can be intrusive (e.g., retinal scanning) or prone to fouling via repeated contact (e.g., fingerprint scanning); (3) Biometric approaches based on optical imaging are expensive, limiting their use to identity checkpoints and major equipment; (4) Identity checkpoints require hardware installed at fixed locations, typically the gates of the facility and the thresholds between areas of differing security levels, so that reconfiguring the security zone layout entails significant renovation; and (5) Identity checkpoints provide only occasional identity confirmation (when the subject attempts passage), rather than continual identity confirmation.

The secure-facility embodiment of the present disclosure can be implemented using a sensor attached on the subject upon entering the facility (e.g., as a bracelet), worn throughout the duration on the premises as ensured by tamper-proof features, and removed upon exiting the facility. The sensor continually confirms the identity and reports the whereabouts of the subject via a wireless link 216. The sensor can also include a direct port for enabling equipment authorized for use by the subject. Alternatively, the sensor could be implemented as a fixed (e.g., wall-mounted) unit at selected doors and gates within the facility.

In one embodiment of the present disclosure, a piezoelectric transducer 235 (e.g., a piezoelectric film) produces an analog signal proportional to the pulse. This can be in place of, or in addition to the optical pulse sensors discussed above. The secure bracelet is placed on the subject and pulse wave characteristics are immediately acquired and stored in a lookup table for future comparison/verification. A analog-to-digital converter transforms the pulse wave to a digital time series. The processor 210 compares the time series to the pulse wave probability distribution stored in local memory.

If the time-series data matches the probability distribution, the processor 210 confirms the subject's identity, and updates the stored probability distribution with new data. If the time series does not match, the processor 210 deems the identity of the test subject to be unconfirmed, and does not update the probability distribution. Either way, the processor 210 can report its decision regarding the test subject's identity to a remote central security manager via a radiofrequency (RF) communications link 216. The processor 210 can also report the test subject's identity to an external device attached to a direct port associated with the present system.

The unit can also include a location sensor (e.g., a GPS unit) in communication with the processor 210. This enables the processor 210 determine the physical location of the subject. For example, the processor 210 can log the subject's path within secure facility, or then trigger an alarm or report to authorities if the subject moves into an unauthorized area. In mobile applications, such as a bracelet or vehicle interlock system, the processor 210 can monitor and communicate the subject's location to authorities via the wireless link 216.

A tamper interlock system 217 detects attempts to remove the sensor, or otherwise prevent its working properly. The tamper system 217 is enabled when a security officer fits the sensor to the subject upon entering the premises, and can only be disarmed by the security officer when the sensor is removed and the subject leaves.

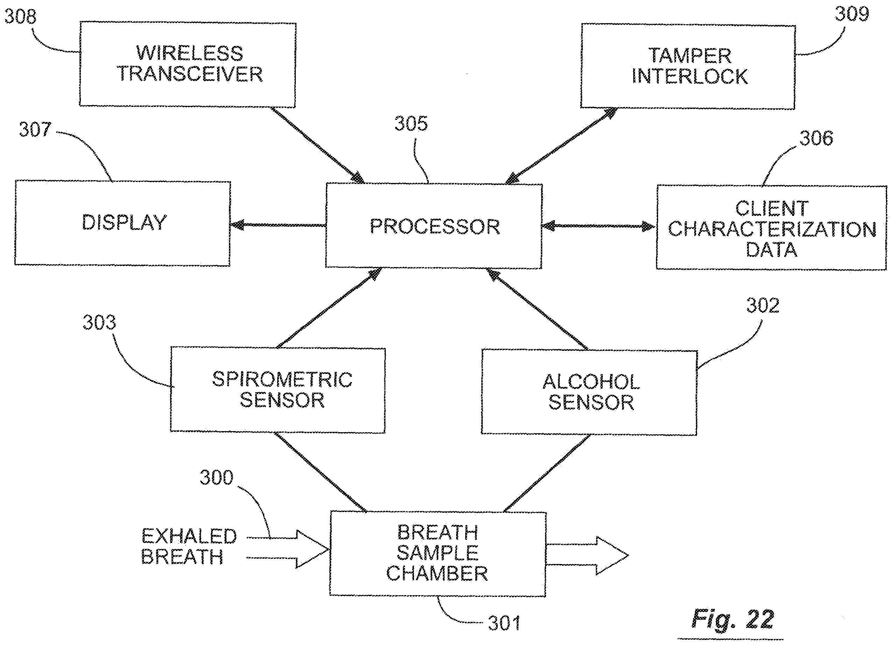

**Biometric Identity Confirmation System Using Spirometric Data.** Yet another unclaimed alternative embodiment of the present disclosure is shown in Figures 22 - 30 that characterizes individuals by spirometric data, for the purpose of identification and identity tracking/confirmation. The first embodiment used the combination of spirometric data and pulse wave data, which provides a significantly higher degree of confidence in subject identification and identity tracking/confirmation. Although providing a somewhat lower degree of confidence, a biometric identity confirmation system based on characterization of spirometric data (without pulse wave data) may be good enough in some fields of use.

Turning to Figure 22, a system block diagram is provided of this embodiment of the present disclosure. The major components include a breath sample chamber 301 for receiving an exhaled breath sample 300 from a client. An alcohol sensor 302 detects the presence of alcohol in the breath sample, and a spirometric sensor 303 measures predetermined spirometric properties of the exhaled breath sample, such as flow or pressure. This spirometric data is typically generated as time series data over the course of the sample. For example, the alcohol sensor 302 can be an off-the-shelf alcohol monitoring component, such as an ethanol-specific electrochemical fuel cell or optical sensor that detects any alcohol present in the breath sample 300.

A computer processor 305 receives and processes data from both of these sensors 302, 303. The system can also include a conventional display 307 controlled by the processor 305, and a wireless transceiver 308 for communication with a remote center for reporting, maintenance and administration. A tamper interlock 309 detects attempts to tamper with the system or otherwise prevent it from working properly. The tamper interlock 309 can cause the processor 305 to trigger a local error / alarm indicator or report the tampering to the remote center via the wireless transceiver 308.

Optionally, the present system can also interface with a vehicle interlock system to prevent a client from operating the vehicle while intoxicated. The present system can include a location sensor (e.g., a GPS unit) in communication with the processor 305. This enables the processor 305 to determine the physical location of the unit and the subject. For example, in mobile application such as a vehicle interlock system, the processor 305 can monitor and communicate the subject's location to authorities via the wireless transceiver 308.

As an overview of operation, the present system requires an initial enrollment mode in which spirometric data for a known client is analyzed to generate client characterization data 306 identifying the known client. Thereafter, in normal operational mode, the processor 305 receives spirometric data from the spirometric sensor 303 for a test client (who may or may not be the known client). The processor 305 analyzes this spirometric data in conjunction with the client characterization data 306 to determine whether the test client is the same as the known client. For the purposes of this application, it should be understood that the phrase "test client" refers to the person whose identity is being tested or confirmed during the operational mode of the present system.

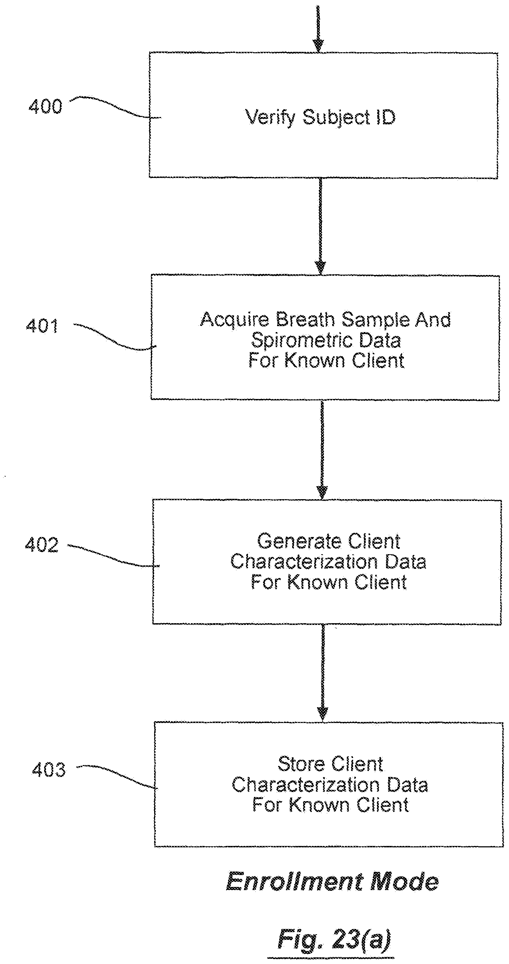

FIG. 23(a) is a flowchart of the enrollment mode employed to initially build client characterization data 306 for a known client. As FVC, FEV₁, and FVL are individual characteristics based on lung volume and respiratory health, the present system will include a client breath characterization process during the enrollment mode. The client will be instructed to take a deep breath and exhale completely into the sampling system. This will be completed a number of times over a period of several minutes with the resulting spirometric data being analyzed and used to generate client characterization data 306. This identifying data will be accessed during each future breath alcohol test for two purposes.

First, the client characterization data file 306 is used to confirm the client is providing a deep lung breath sample based on their capabilities. It is important to note that this feature is a significant advancement upon current breath alcohol testing devices. The current state of the art is to measure a minimum average threshold value to validate a test. With human lung capacity ranging from 1.5 to 6 liters, the opportunity for error is obvious.

Second, the client characterization data 306 is used to confirm the client is providing his own sample (i.e., to verify client identity of an in-person or a remote alcohol test). While colds and flu can create some variability, the client's lung capacity is an individual characteristic and is relatively constant over a period of months.

In particular, during enrollment in Figure 23(a), the operator first verifies the identity of the known client (step 400). The processor 305 acquires spirometric data from the spirometric sensor 303 for a number of sample periods (step 401). The processor 305 analyzes the enrollment spirometric data and generates client characterization data 306 for identifying the known client (step 402). This client characterization data 306 is stored for later use during the operational mode of the present system (step 403) as will be described below.

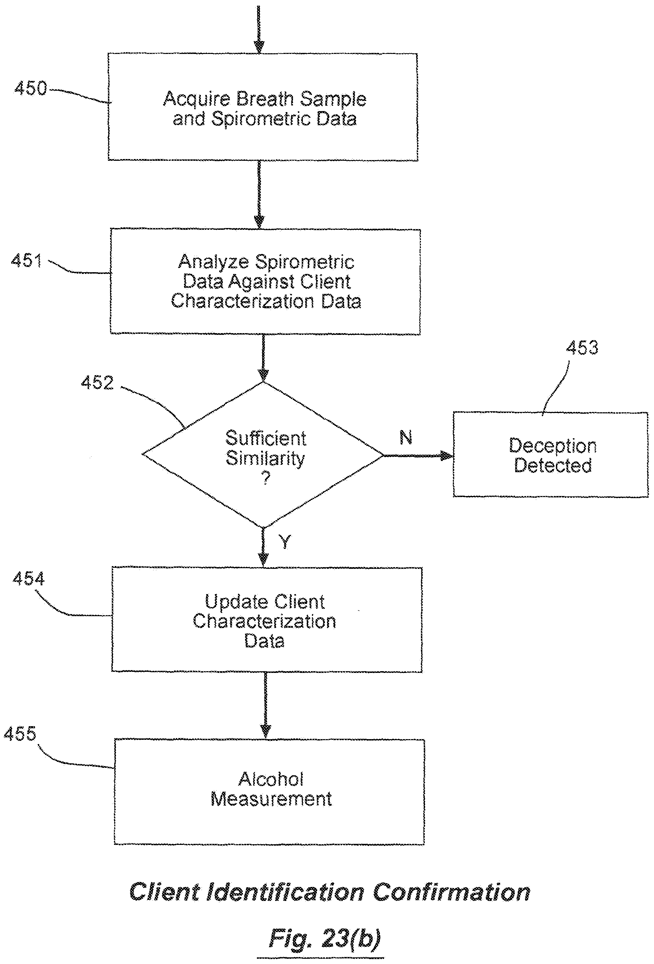

Following completion of the enrollment mode, the present system proceeds to operational mode of client identity confirmation during day-to-day monitoring of the client. FIG. 23(b) is a flowchart of this operational mode. During each breath alcohol test, the processor 305 acquires spirometric data from the spirometric sensor 303 as the test client exhales a breath sample 300 into the breath sample chamber 301 (step 450). The processor 305 analyzes this spirometric data using the client characterization data 306 (step 451). Based on this analysis, the processor 305 determines whether there is a sufficient degree of similarity between the spirometric characteristics of the known client (from the client characterization data 306) and the test client to conclude that these are the same person (step 452). If so, the processor 305 may update the client characterization data 306 to include the current spirometric data (step 454) and then proceed with alcohol measurement in the breath sample (step 455). Otherwise, if the processor 305 determines that the current test client is not the same as the known client, an alarm can be activated to signal that deception has been detected (step 453). The processor 305 can also remotely alert the authorities via a wireless transceiver 308, or store results in a local log for later retrieval by the system administrator.

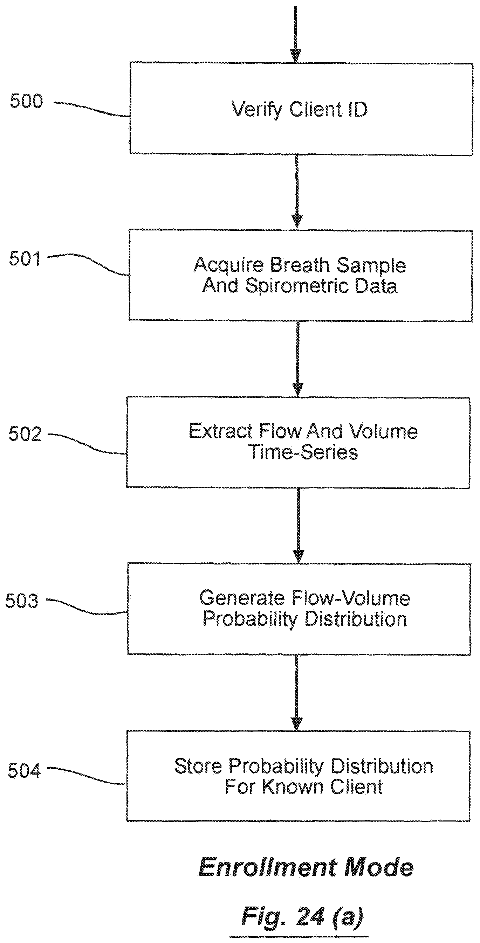

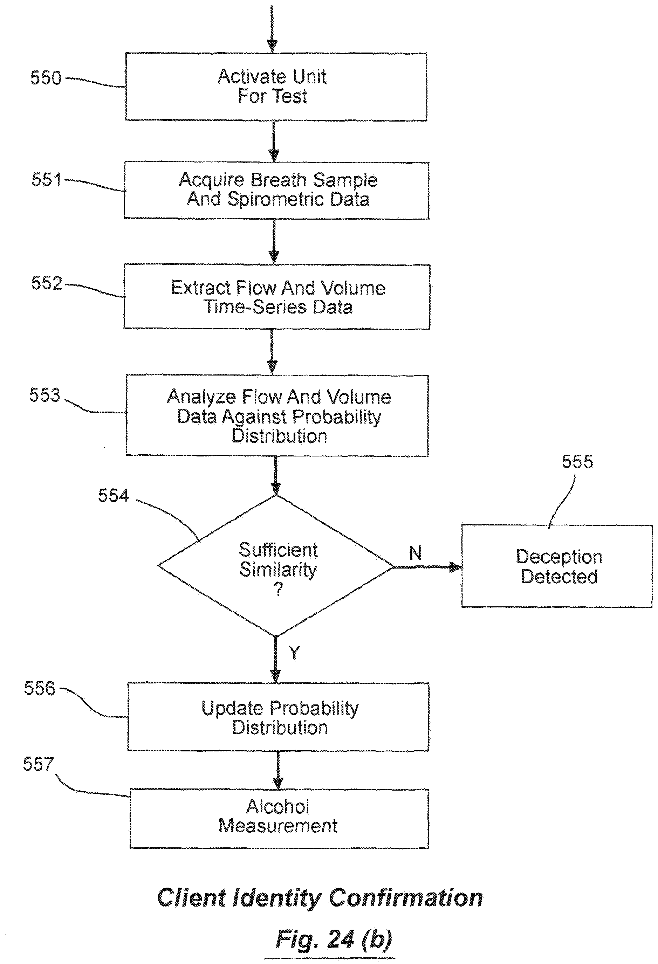

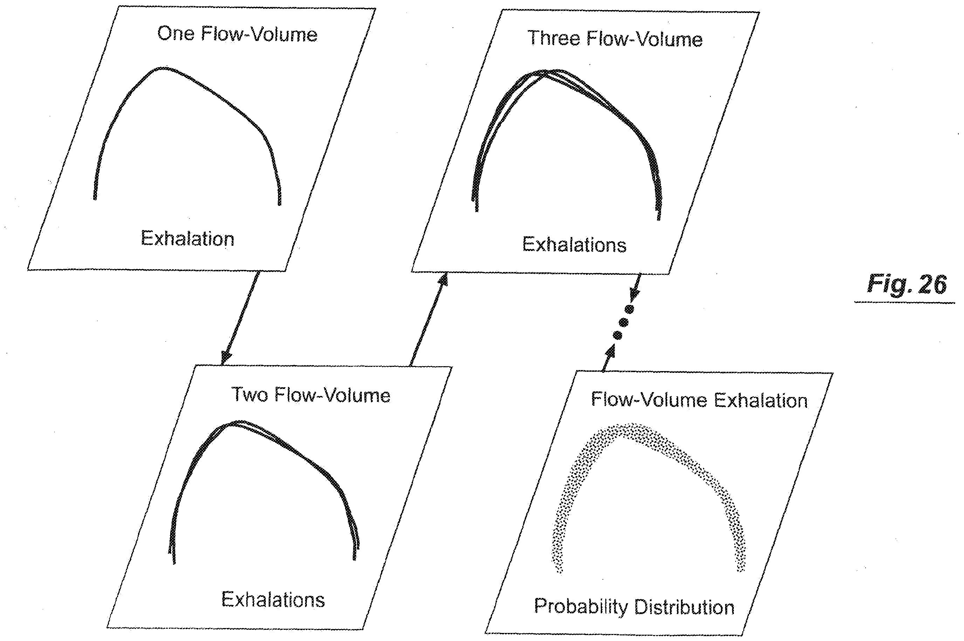

Figures 24(a) and 24(b) are flowcharts of the enrollment mode and operational mode, respectively, for an embodiment of the present disclosure in which the client characterization data 306 is stored as a probability distribution in two-dimensional phase space. As an example, the client characterization data 306 might be formulated as a learned probability density in flow-volume space. Because just exhalation is measured, only half the FVL is characterized. A strictly repeating phenomenon will trace the same sharp curve in flow-volume space over and over, while a measurement that varies from cycle to cycle will yield a blurred, probabilistic curve, as shown for example in Figure 26. Thus, spirometric data from a number of breath samples are typically necessary to complete the enrollment process.

There are several advantages to the learned-probability approach. First, it relies on no artificial or simplistic assumptions about the dynamics, as do models. Second, it utilizes all data, rather than heavily weighting prominent features such as peak expiratory flow (PEF). Third, it naturally yields the optimal decision and probability of error in detecting identity deception.

A client's FVL probability density is acquired during the enrollment period, as generally discussed above with regard to Figures 23(a) and 23(b). In this embodiment, the FVL data from multiple breath samples for the client are combined and stored as a probability distribution in phase space. This probability distribution serves as the client characterization data 306, discussed above.

In general terms a "phase space" is a multi-dimensional (D-dimensional) space in which a spirometric variable is correlated with (D-1) other measurements. The other measurements can be the same variable measured at various times in the past, or other contemporary variables, or a combination. The D measurements form a vector that traces an "orbit" or repeating pattern in phase space over a series of breath samples. A strictly periodic phenomenon will follow the same orbit over and over, and will soon be utterly predictable. A phenomenon that varies from breath sample to sample will yield a blurred, probabilistic orbit.

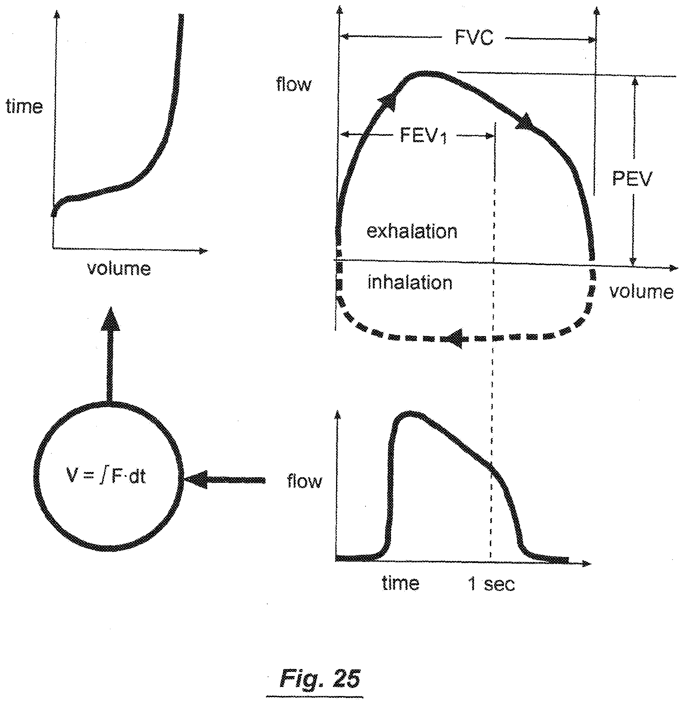

This specific embodiment employs flow and volume time-series data as the variables in the probability distribution. Figure 25 depicts the reduction of flow and volume time-series into a flow versus volume orbit in phase space. Figure 26 is a diagram illustrating how a flow-volume probability distribution can be built up over many breath samples.

The phase space domain is usually just the outer product of its scalar variable domains. With 8-bit analog-to-digital conversion (ADC), a 2-D phase space needs but a modest 65,536-address memory. An 8-bit ADC is probably adequate, considering the small dynamic range and noisiness of the signals. The phase space range should be appropriate for storing a probability - for instance, an unsigned integer. Since the orbit visits some phase space cells much more frequently than others, the integer must have sufficient dynamic range, say 16 bits. Thus, the example phase space probability density memory requirement is 128 kilobytes. In other words, the phase space is effectively a 2-D array of cells or elements, each of which store an integer value representing the probability associated with a particular pair of flow and volume values for the client. This phase space can be referred to as a flow-volume probability distribution.

Returning to the enrollment mode in Figure 24(a), the identity of a known client is first verified in step 500. This client is then required to blow into the sensor a number of times in the presence of a technician so that several sets of spirometric data can be acquired (step 501). The spirometric data is then analyzed to extract flow and volume time-series data (step 502). The flow data and volume data can then be combined to generate a FVL (step 503), which is stored as a probability distribution (step 504) for later use in the operational mode.

After enrollment, the system can be used in its operational mode. FIG. 24(b) is a flowchart of the operational mode for this embodiment. Prior to each breath test, the unit is first activated by the client (step 550). For example, this can be done by activating a switch, or by sensing contact with the client's lips, or by pressure exerted by the client's exhalation into the unit. During the breath sample, the processor 305 acquires spirometric time-series data from the spirometric sensor 303 for the test client (step 551). The processor 305 converts this raw data into flow and volume time-series data (step 552). The flow-volume probability distribution serves as a look-up table for the probability associated with each pair of flow and volume values measured during operational mode. In particular, the processor 305 retrieves from the flow-volume probability distribution the probability associated with the pairs of flow and volume values. Analysis of the probabilities associated with the set of pairs of flow and volume values enables the processor 305 to determine whether there is a sufficient similarity between the spirometric characteristics of the known client and test client currently using the unit (step 553).

The probability density can be quite specific to a client, without assuming any particular model. Deception is detected when the compound probability of measuring the latest N data is deemed sufficiently small (step 554). More specifically, a deception is judged when the cost of erroneously regarding the subject as truthful exceeds the cost of erroneously regarding the subject as deceptive: C(t|D)×P(D|M) > C(d|T)×P(T|M), where C(t|D) is the penalty for judging the subject truthful when in fact deceptive, and P(D|M) is the (unknown) conditional probability of deception given the measurement M, and vice versa for the right-hand side of the inequality.

Bayes' Theorem states: P(D|M)×P(M) = P(D,M) = P(M|D)×P(D), where P(M) is the (inconsequential) a priori probability of measuring M, P(D,M) is the (undesired) joint probability of deception and measuring M, P(M|D) is the (known) conditional probability of measuring M given deception, and P(D) is the (estimated) probability of deception. Substituting into the cost condition and rearranging gives P(M|D)/P(M|T) × P(D)/P(T) > C(d|T)/C(t|D). These factors are all known or estimated: P(M|D) is given by the average of all subjects' probability densities, assuming this average represents the general population, and the subject is as likely to pass off the sensor to anyone as to anyone else; P(M|T) is given by the subject's own probability density; P(D) is estimated from a subject's past behavior (e.g., a subject who has not attempted deception in three tests per day for a month has on the order of 1% chance of attempting deception on the next test); P(T) is merely 1-P(D); and C(d|T) and C(t|D) are input parameters.

The specificity of client identity confirmation can be quantified by the probability of the client successfully colluding with a random impostor. As previously noted, the FVL (encompassing the FVC, FEV1, PEF and possibly other spirometric parameters) must be at least as specific as the parameter vector (FVC, FEV1, PEF,...). Thus, the specificity estimated for the parameter vector is pessimistic, and the actual FVL specificity may be better. For most of the population, FVC and FEV1 each span approximately a factor of two. Since repeatability is typically about 5%, a random member of the population can be assigned to one of about ten classes for each of FVC and FEV1. PEF is strongly correlated with FEV1, but since it's a peak rather than an integrated measure, it's likely noisier. Therefore, PEF is not considered in this analysis.

Suppose FVC and FEV1 each span ten distinguishable classes. Using either by itself yields a 10% probability of successful deception. Were FVC and FEV1 fully independent, and matching both were required, the probability of successful deception drops to 1%. If FVC and FEV1 were perfectly correlated, checking either is as good as checking both, and the probability of successful deception remains about 10%. The geometric mean, about 3% is a realistic expectation. Thus, the combination of FVC and FEV1 for client identity confirmation will false-negative (report all is well, when in fact an impostor has supplied the breath sample) about one time in thirty.

Since performance is limited from above by mechanical and physiological constraints, breath tests resulting in the largest FVLs are most trustworthy. The greatest or greatest few measurements initialize the probability density. Enrollment serves the concomitant function of training the subject to put forth a maximal effort. After enrollment, and during normal operation, new data deemed genuine updates the probability density (step 556). In order to weight new data more than old data, and to prevent overflow, the accumulated probability density is continuously devalued. On the other hand, if data is deemed bogus, the system can alert the authorities to possible deception (step 555). Bogus data should obviously not be allowed to corrupt the probability density for the subject. Assuming the identity of the test client is validated, the system can proceed with measurement of any alcohol in the breath sample (step 557).

Another embodiment of the present disclosure relies on statistical analysis of a plurality of spirometric parameters for each client. In other words, the client characterization data includes a plurality of spirometric parameters, such as FVC, PEF and FEV1. Data from a statistically significant set of breath samples can be acquired and analyzed during the initial enrollment period and also during the subsequent operational mode to determine mean and standard deviation values for each of these spirometric parameters. The use of a combination of multiple spirometric parameters increases the confidence of a correct identification of a client.

In yet another embodiment of the present disclosure the shape of the flow curve during the expiratory phase can be characterized by a number parameters. In particular, a typical flow versus time curve is generally trapezoidal consisting of the following stages. First, there is a rapid onset with flow increasing over a few tenths of a second from zero to PEF at the beginning of exhalation. Next, there is a gradual diminution in flow over several seconds during exhalation, which can be characterized by a slope, dF/dt, and possibly a curvature parameter. Finally, there is a rapid decrease in flow to zero (or "collapse"), when no more air is exhaled.

For example, this type of analysis can yield the following parameters: (1) PEF - technically, the largest flow value in the data set, but a more repeatable proxy for Fmax, the intercept of a linear least-squares fit to the droop-stage data; (2) dF/dt - the slope of the least-squares fit to the droop-stage data; and (3) FVC - the time integral of flow over all three stages. Other possible sets of spirometric parameters include Vmax, Fmax and dF/dt. Here again, means and standard deviation values can be calculated and stored for each of these spirometric parameters.

In some cases, the use of a trapezoidal paradigm may be too simplistic. Some breath profiles show a substantial roundedness and are better modeled by quadratic or polynomial curve fitting. In this embodiment, the resulting coefficients from quadratic or polynomial curve fitting, together with FVC, could serve as the spirometric parameters.

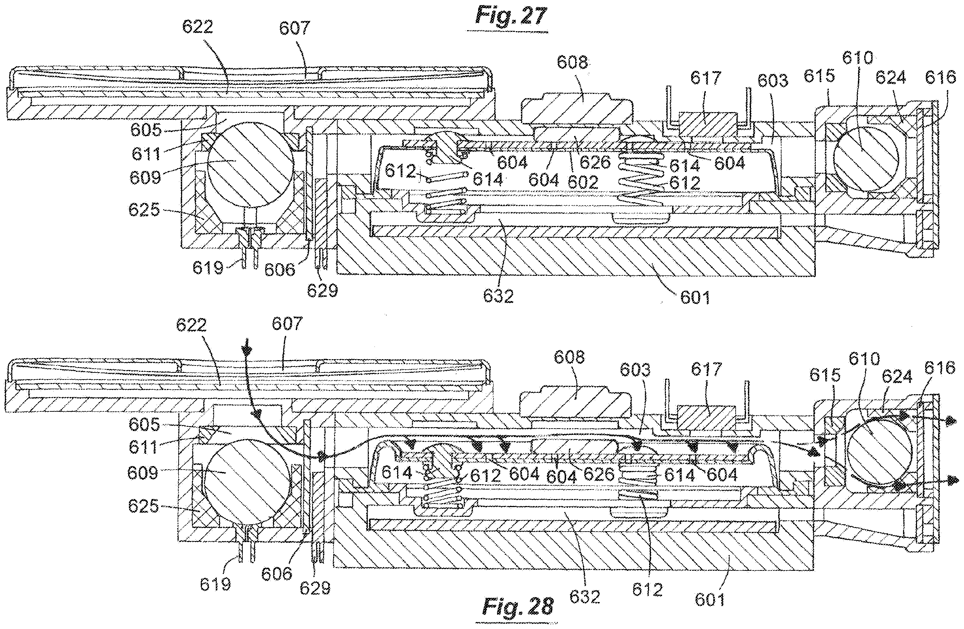

The breath sampling system depicted in the cross-sectional views provided in Figure 27 -30 illustrates one possible physical embodiment of the present disclosure. This system offers several key advancements and innovations in the field of breath alcohol testing. First, the system has only passive mechanical components, without electrically-powered pumps or valves. Sampling is powered solely by the mechanical energy of exhalation. The system has only four moving parts, thereby providing excellent mechanical reliability. In contrast, many conventional breath sampling devices employ a mechanical pump, which increase power requirements and are often prone to failure.

Second, the present system ensures a deep lung breath sample is transferred to the breath alcohol concentration sensor to avoid spuriously low or high readings. Third, the present system provides spirometric client identity confirmation (CIC). Finally, the only active components are a temperature sensor for breath temperature compensation, an infrared proximity sensor/diaphragm for measuring airflow, and an interrupt mechanism for detecting the commencement and cessation of exhalation. It should be understood that other types of pressure or flow sensors may be substituted for the diaphragm deflection / proximity sensor arrangement. The present system can be is enclosed in a compact housing.

In this embodiment of the present disclosure, the sampling procedure and the breath alcohol concentration analyzer hardware dovetail together well. An accurate breath alcohol measurement requires an air sample from deep within the lungs, essentially the tail end of a maximal exhalation. The usual strategies for subverting a breath alcohol test - reserving exhalation and counterfeiting the sample - are precisely those the present disclosure is designed to foil.

Figure 27 is a cross-sectional view of the breath alcohol testing device in its initial locked state (i.e., prior to a breath test). A magnetic coil 608 on the housing of the unit attracts a small permanent magnet 626 attached to the diaphragm 602, so that the diaphragm 602 is held in place against the interior of the unit housing to prevent damage during transportation or storage of the unit, and to assure a uniform starting position of the diaphragm.

Figure 28 is a cross-sectional view of the breath alcohol testing device in its activated state at the beginning a breath alcohol test. The magnetic coil 608 releases the magnet 626 to allow the diaphragm 602 to move during the testing process. In operation, the subject presses his lips against the lip contact plate 607, and blows into the entry chamber 605. The breath traverses a porous hydrophobic membrane 622, and its positive pressure dislodges the metal ball valves 609 and 610 from their respective magnetic washers 611 and 615 into ball valve cages 625 and 624, as shown in Figure 28. This allows the breath gases to flow from the inlet ball valve 609 through a secondary membrane 606 into an upper sample chamber 603, and then exit at the outlet ball valve 610 through an exhaust membrane 616.

The pressure generated by blowing separates the inlet ball valve 609 from its magnetic washer 611 and makes contact with activation contacts 619 This closes a low-power circuit causing an interrupt to awake a processor and inform it that a breath alcohol test is taking place. The processor then reads a baseline from the breath alcohol concentration sensor (fuel cell 601) and reads the temperature of the breath via a temperature sensor 629. The processor also reads the deflection of the diaphragm 602 via an infrared sensor 617 to assure it is depressed by positive pressure, and initiates a counter.

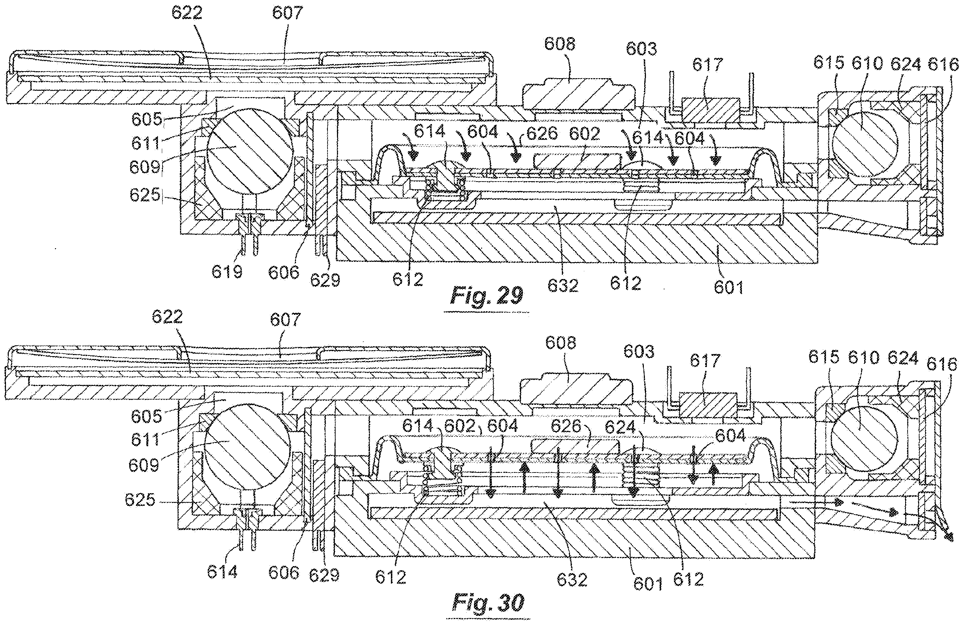

The flow restriction provided by the outlet ball valve 610 serves as a known resistance, which creates back pressure within the upper sample chamber 603. As this pressure builds, the diaphragm 602 is depressed toward the base of the fuel cell sample chamber 632, as shown in Figure 29. Repeatable resistance to this downward movement of the diaphragm 602 is provided by a number of compression springs 612. Any air in the fuel cell sample chamber 632 is purged through the exhaust port 621 as the diaphragm 602 is incrementally depressed. During exhalation, an infrared sensor 617 measures the diaphragm 602 deflection at predetermined intervals. Due to the known resistance of the outlet ball valve 610, changes in the position of the diaphragm are proportional to changes in pressure within the sample chamber. The processor 305 can then calculate total breath flow based on diaphragm deflection versus time over the sampling period.

Temperature readings can be utilized to calibrate both the spirometric measurements and the reported breath alcohol concentration; and to confirm the sample is in the 30°C to 37°C range expected of a human test subject, as part of client identity confirmation. The counter will time the duration of flow through the fuel cell sample chamber 632, starting with the interrupt generated by breaking interrupt contacts 619, and stopping when exhalation finishes, remaking contact. This informs the microprocessor that breath sampling has ended, and the deep lung breath alcohol sample is now in the external sample chamber.

Upon the reduction in pressure as exhalation ends, the metal ball valves 609 and 610 are attracted to the magnetic washers 611 and 615, returning to their original closed and sealed position, as shown in Figure 30. Compression springs 612 mounted to rivets 614 under the diaphragm 602, having been compressed by the back pressure of exhalation on the flow restriction provided by the outlet ball valve 610, gradually move the diaphragm 602 upward to its quiescent position. As this occurs, the breath in the upper sample chamber 603 is forced through a number of small-diameter holes 604 or reed valves in the face of diaphragm 602. The small-diameter holes 604 allow a calibrated breath sample to enter the fuel cell's internal sample chamber 632 .

The breath sample is presented to the breath alcohol concentration sensor (e.g., a fuel cell 601 that oxidizes ethanol and converts it to a proportional electrical signal). Any ethanol measurement sensor may be substituted, particularly infrared measurement systems. The processor 305 then computes the breath alcohol concentration using the fuel cell output, breath sample temperature, fuel cell temperature, and various calibration factors.

The present disclosure could also employ a mechanical flow meter piggybacking on an existing breath alcohol sampler hardware design, providing an exhaled air-flow time series to the processor 305. Alternatively, air flow can be inferred from the pressure drop across a calibrated orifice. The air flow may be time-integrated to yield the FVC, thus also providing the spirogram and FVL.

The above disclosure sets forth a number of embodiments of the present invention described in detail with respect to the accompanying drawings. Those skilled in this art will appreciate that various changes, modifications, other structural arrangements, and other embodiments could be practiced under the teachings of the present invention without departing from the scope of this invention as set forth in the following claims.

## Claims

1. A method for biometric identity confirmation of a subject having a pulse and a respiratory cycle, said method comprising:
during an initial training mode,
simultaneously acquiring pulse waveform data and spirometric data from a known subject;
generating (123) and storing (124) subject characterization data for the known subject derived at least in part from both the pulse waveform data and spirometric data for the known subject; and
during a subsequent identity authentication mode,
simultaneously acquiring pulse waveform data and spirometric data from a test subject (125), and
analyzing the pulse waveform data and spirometric data with the subject characterization data for the known subject to confirm whether the identity of the test subject matches the known subject (126);
wherein in both the training and identity authentication modes,
acquiring the spirometric data includes the subject exhaling into a testing unit (10) and exerting labial pressure on a mouthpiece (15) of the testing unit (10), and
acquiring the pulse waveform data includes simultaneously performing lip pulse photoplethysmography.

2. The method of claim 1 wherein the subject characterization data is derived at least in part from the first derivative of the pulse waveform data with respect to time.

3. A method as claimed in claim 1 or 2, wherein the subject characterization data for the known subject is derived at least in part from analysis of both the pulse waveform data and spirometric data for the known subject, said analysis including
measurement of characteristic changes in the pulse waveform data as a function of the phase of the respiratory cycle of the known subject.

4. The method of any one of the preceding claims, wherein the subject characterization data comprises the forced vital capacity (FVC).

5. The method of any one of the preceding claims, wherein the subject characterization data comprises the peak expiratory flow (PEF).

6. The method of any one of the preceding claims 4, wherein the subject characterization data comprises the forced expiratory volume in one second (FEV₁).

7. The method of any of the preceding claims, wherein generating the subject characterization data in the training mode, and acquiring and analyzing the pulse waveform data in the identity authentication mode include the pulse waveform data comprising a plurality of pulse wave cycles, and include the processor
querying a pulse sensor at intervals to sample the pulse wave and provide digitized time series (130);
calculating the first and second derivatives of the pulse waveform data with respect to time, to provide respective first and second derivative time series (131);
identifying pulse wave cycle triggers in the second derivative time series, the triggers indicating consistent start points for the systolic excursion;
parsing the pulse waveform data into synchronized pulse wave cycles, using the triggers the synchronize the pulse wave cycles (132); and ave
raging corresponding points in the synchronized first derivative time series (133).

8. The method of any of the preceding claims, in which generating the subject characterization data in the training mode, and acquiring and analyzing the pulse waveform data in the identity authentication mode include:
simultaneously performing tonometry and photoplethysmography on the subject, to provide the pulse waveform data comprising simultaneously measured blood pressure and pulsatile blood volume of the subject; and the processor
determining a Womersley number for the subject.

## Patentansprüche

1. Verfahren zur biometrischen Identitätsbestätigung eines Subjekts mit einem Puls und einem Atemzyklus, wobei das Verfahren Folgendes umfasst:
während eines anfänglichen Trainingsmodus,
gleichzeitiges Erfassen von Pulswellenformdaten und spirometrischen Daten von einem bekannten Subjekt;
Erzeugen (123) und Speichern (124) von Subjektkennzeichnungsdaten für das bekannte Subjekt, die mindestens teilweise sowohl aus den Pulswellenformdaten als auch aus den spirometrischen Daten für das bekannte Subjekt abgeleitet werden; und
während eines folgenden Identitätsauthentifizierungsmodus,
gleichzeitiges Erfassen von Pulswellenformdaten und spirometrischen Daten von einem Testsubjet (125), und
Analysieren der Pulswellenformdaten und der spirometrischen Daten mit den Subjektkennzeichnungsdaten für das bekannte Subjekt, um zu bestätigen, ob die Identität des Testsubjekts mit dem bekannten Subjekt übereinstimmt (126);
wobei sowohl in dem Trainings- als auch in dem Identitätsauthentifizierungsmodus
das Erfassen der spirometrischen Daten beinhaltet, dass das Subjekt in eine Testeinheit (10) ausatmet und mit den Lippen Druck auf ein Mundstück (15) der Testeinheit (10) ausübt, und
das Erfassen der Pulswellenformdaten das gleichzeitige Durchführen einer Lippen-Puls-Photoplethysmographie beinhaltet.

2. Verfahren nach Anspruch 1, wobei die Subjektkennzeichnungsdaten mindestens teilweise aus der ersten Ableitung der Pulswellenformdaten in Bezug auf die Zeit abgeleitet werden.

3. Verfahren nach Anspruch 1 oder 2, wobei die Subjektkennzeichnungsdaten für das bekannte Subjekt mindestens teilweise aus der Analyse sowohl der Pulswellenformdaten als auch der spirometrischen Daten für das bekannte Subjekt abgeleitet werden, wobei die Analyse Folgendes beinhaltet
Messung von Kennzeichnungsänderungen in den Pulswellenformdaten in Abhängigkeit der Phase des Atemzyklus des bekannten Subjekts.

4. Verfahren nach einem der vorhergehenden Ansprüche, wobei die Subjektkennzeichnungsdaten die forcierte Vitalkapazität (FVC) umfassen.

5. Verfahren nach einem der vorhergehenden Ansprüche, wobei die Subjektkennzeichnungsdaten den exspiratorischen Spitzenfluss (PEF) umfassen.

6. Verfahren nach einem der vorhergehenden Ansprüche 4, wobei die Subjektkennzeichnungsdaten das forcierte exspiratorische Volumen in einer Sekunde (FEV₁) umfassen.

7. Verfahren nach einem der vorhergehenden Ansprüche, wobei das Erzeugen der Subjektkennzeichnungsdaten in dem Trainingsmodus und das Erfassen und Analysieren der Pulswellenlängendaten in dem Identitätsauthentifizierungsmodus beinhaltet, dass die Pulswellenformdaten eine Vielzahl von Pulswellenzyklen umfassen, und beinhaltet, dass der Prozessor
einen Pulssensor in Intervallen abfragt, um die Pulswelle abzutasten und eine digitalisierte Zeitreihe bereitzustellen (130) ;
die erste und die zweite Ableitung der Pulswellenformdaten in Bezug auf die Zeit berechnet, um eine jeweilige erste und zweite Ableitungszeitreihe bereitzustellen (131);
Pulswellenauslöser in der zweiten Ableitungszeitreihe identifiziert, wobei die Auslöser einheitliche Anfangspunkte für die systolische Auslenkung angeben;
die Pulswellenformdaten in synchronisierte Pulswellenzyklen zerlegt, wobei die Auslöser zum Synchronisieren der Pulswellenzyklen (132) verwendet werden; und
die entsprechenden Punkte in der synchronisierten ersten Ableitungszeitreihe mittelt (133).

8. Verfahren nach einem der vorhergehenden Ansprüche, bei dem
das Erzeugen der Subjektkennzeichnungsdaten in dem Trainingsmodus und das Erfassen und Analysieren der Pulswellenformdaten in dem Identitätsauthentifizierungsmodus Folgendes beinhaltet:
gleichzeitiges Durchführen von Tonometrie und Photoplethysmographie an dem Subjekt, um die Pulswellenformdaten bereitzustellen, welche den Blutdruck und das Pulsationsblutvolumen des Subjekts, die gleichzeitig gemessen werden, umfassen; und wobei der Prozessor eine Womersley-Zahl für das Subjekt bestimmt.

## Revendications

1. Procédé pour une confirmation d'identité biométrique d'un sujet présentant un pouls et un cycle respiratoire, ledit procédé comprenant :
pendant un mode d'entraînement initial,
l'acquisition simultanée de données de forme d'onde de pouls et de données spirométriques provenant d'un sujet connu ;
la génération (123) et le stockage (124) de données de caractérisation de sujet pour le sujet connu dérivées au moins en parties des données de forme d'onde de pouls et des données spirométriques pour le sujet connu ; et
pendant un mode d'authentification d'identité ultérieur,
l'acquisition simultanée de données de forme d'onde de pouls et de données spirométriques provenant d'un sujet test (125), et
l'analyse des données de forme d'onde de pouls et des données spirométriques avec les données de caractérisation de sujet pour le sujet connu pour confirmer si oui ou non l'identité du sujet test correspond au sujet connu (126) ;
dans lequel dans les modes d'entraînement et d'authentification d'identité,
l'acquisition des données spirométriques comprend l'expiration du sujet dans une unité de test (10) et l'exercice d'une pression labiale sur un embout buccal (15) de l'unité de test (10), et
l'acquisition des données de forme d'onde de pouls comprend la réalisation simultanée d'une photopléthysmographie de pouls au niveau des lèvres.

2. Procédé selon la revendication 1 dans lequel les données de caractérisation de sujet sont dérivées au moins en partie de la première dérivée des données de forme d'onde de pouls par rapport au temps.

3. Procédé selon la revendication 1 ou 2, dans lequel les données de caractérisation de sujet pour le sujet connu sont dérivées au moins en partie d'une analyse des données de forme d'onde de pouls et des données spirométriques pour le sujet connu, ladite analyse comprenant
une mesure de changements caractéristiques des données de forme d'onde de pouls en fonction de la phase du cycle respiratoire du sujet connu.

4. Procédé selon l'une quelconque des revendications précédentes, dans lequel les données de caractérisation de sujet comprennent la capacité vitale forcée (FVC).

5. Procédé selon l'une quelconque des revendications précédentes, dans lequel les données de caractérisation de sujet comprennent le débit maximal respiratoire (PEF).

6. Procédé selon l'une quelconque des revendications précédentes 4, dans lequel les données de caractérisation de sujet comprennent le volume expiratoire forcé en une seconde (FEV₁).

7. Procédé selon l'une quelconque des revendications précédentes, dans lequel la génération des données de caractérisation de sujet dans le mode d'entraînement, et l'acquisition et l'analyse des données de forme d'onde de pouls dans le mode d'authentification d'identité comprennent les données de forme d'onde de pouls comprenant une pluralité de cycles d'onde de pouls, et comprennent les opérations suivantes réalisées par le processeur
l'interrogation d'un capteur de pouls à intervalles pour échantillonner l'onde de pouls et fournir une série chronologique numérisée (130) ;
le calcul des première et seconde dérivées des données de forme d'onde de pouls par rapport au temps, pour fournir des première et seconde séries chronologiques dérivées respectives (131) ;
l'identification de déclencheurs de cycle d'onde de pouls dans la seconde série chronologique dérivée, les déclencheurs indiquant des points de départ cohérents pour l'excursion systolique ;
l'analyse des données de forme d'onde de pouls en cycles d'onde de pouls synchronisés, en utilisant les déclencheurs pour synchroniser les cycles d'onde de pouls (132) ; et
le calcul de la moyenne de points correspondants dans la première série chronologique dérivée synchronisée (133).

8. Procédé selon l'une quelconque des revendications précédentes, dans lequel
la génération des données de caractérisation de sujet dans le mode d'entraînement, et l'acquisition et l'analyse des données de forme d'onde de pouls dans le mode d'authentification d'identité comprennent :
la réalisation simultanée d'une tonométrie et d'une photopléthysmographie sur le sujet, pour fournir les données de forme d'onde de pouls comprenant la pression artérielle et le volume sanguin pulsatile du sujet mesurés simultanément
et la détermination par le processeur d'un nombre de Womersley pour le sujet.
